# EUROPÄISCHE PATENTANMELDUNG

(11) **EP 1 430 933 A2**
(43) Veröffentlichungstag der Anmeldung: **23.06.2004**
(21) Anmeldenummer: 03028617.3
(22) Anmeldetag: 11.12.2003
(51) Int. Cl.: A61P 17/14, A61K 31/185, A61K 31/05, A61K 31/19, A61K 31/191, A61K 31/195, A61K 31/198, A61K 31/695, A61K 31/6615, A61K 31/7004, A61K 7/06

(54) **Kosmetische und pharmazeutische Zusammensetzungen enthaltend Steroidsulfatase-Inhibitoren und deren Verwendung zur Verminderung von Haarausfall**

(30) Priorität: 20.12.2002 DE 10260955
(71) Anmelder: Henkel Kommanditgesellschaft auf Aktien, 40589 Düsseldorf (DE)
(72) Erfinder: Siegert, Petra,, 42781 Haan (DE); Banowski, Bernhard, 40597 Düsseldorf (DE)

(57) **Zusammenfassung**

Gegenstand der Erfindung ist die Verwendung von ausgewählten Steroidsulfatase-inhibierenden Substanzen in kosmetischen oder pharmazeutischen Zusammensetzungen zur Verminderung von Haarausfall.

## Beschreibung

Gegenstand der Erfindung ist die Verwendung von ausgewählten Steroidsulfatase-inhibierenden Substanzen in kosmetischen oder pharmazeutischen Zusammensetzungen zur Verminderung von Haarausfall.

Haarausfall bei Männern, aber auch bei Frauen, wird am häufigsten durch die Einwirkung von Steroidhormonen verursacht. Es ist bekannt, dass der Haarausfall in Zusammenhang mit dem Steroidstoffwechsel, bei Männern insbesondere mit einer vergleichsweise hohen Testosteronausschüttung, steht. Das Testosteron wird durch die 5-α-Reduktase zu 5-α-Dihydrotestosteron (5a-DHT) umgewandelt, welches eine entscheidende Rolle bei der Regulation des Haarwachstums spielt. Androgensensitive Haarfollikel (wie insbesondere das vordere Haupthaar bei Männern) zeigen bei erhöhter 5a-DHT-Konzentration eine Schrumpfung des Haarfollikels, welche zu fortschreitend dünner und kürzer werdenden Haaren bis zum Haarausfall führt. Es konnte gezeigt werden, dass Inhibitoren der 5-α-Reduktase (wie Finasterid®)zu einer Verminderung des Haarausfalls führen können.

In dem Artikel "Enzymology of the hair follicle", R.Hoffmann, Eur. J. Dermatol. 2001, Vol. 11, S. 296-300, werden als weitere mögliche Stoffwechselwege zur Bildung von DHT die Umsetzungen von Vorläufern wie Dehydroepiandrosteronsulfat zu DHT beschrieben, die z.T. nicht Testosteron als Vorstufe von DHT umfassen. Inhibitoren der 5-α-Reduktase sind daher möglicherweise allein nicht in der Lage, die Bildung von DHT zu vermindern.

Aufgabe der vorliegenden Erfindung ist es daher, alternative Stoffe bereits zu stellen, die Haarausfall, insbesondere den androgenetischen Haarausfall vermindern können.

Gegenstand der vorliegenden Erfindung ist die Verwendung von mindestens einer Steroidsulfatase-inhibierenden Substanz, ausgewählt aus funktionellen C₂-C₁₂-Carbonsäuren und deren physiologisch verträglichen Metallsalzen, Derivaten von C₃-C₉-Polyolen, Phenolderivaten, Pflanzenextrakten, Riechstoffen, Flavonoiden, Aluminiumchlorohydrat, 2-Phenylethanol, Etidronsäure, 7-Acetyl-1,1,3,4,4,6-hexamethyltetralin, Tropolonderivaten, Estern der Schwefelsäure mit alkoxylierten C₈-C₁₈-Fettalkoholen und deren physiologisch verträglichen Metallsalzen, Estern der Phosphorsäure und der Triphosphorsäure mit ein- bis sechswertigen Hydroxyverbindungen, Kieselsäureestern, aus marinen Organismen isolierbaren mycosporinähnlichen Aminosäuren (MAA) sowie quaternären Siliconverbindungen in einer kosmetischen oder pharmazeutischen Zusammensetzung zur Verminderung des Haarausfalls.

Erfindungsgemäß bevorzugt ist die Verwendung von AluminiumchlorohydratenFür die Inhibierung von Steroidsulfatase genügen niedrigere Konzentrationen von 0,2 bis 3 Gew.-%, bevorzugt 0,5 bis 3 Gew.-% und besonders bevorzugt 1,0 bis 2,0 Gew.-%, jeweils bezogen auf die gesamte kosmetische oder pharmazeutische Zusammensetzung.
Erfindungsgemäß wird Aluminiumchlorohydrat in Mengen von 0,1 bis 10 Gew.-%, bevorzugt 0,5 bis 5,0 und besonders bevorzugt 1,0 bis 2,0 Gew.-%, jeweils bezogen auf die gesamte kosmetische Zusammensetzung, eingesetzt.

Erfindungsgemäß wird 2-Phenylethanol in Mengen von 0,1 bis 10 Gew.-%, bevorzugt 0,5 bis 5,0 und besonders bevorzugt 1,0 bis 2,0 Gew.-%, jeweils bezogen auf die gesamte kosmetische Zusammensetzung, eingesetzt. Erfindungsgemäß wird Etidronsäure in Mengen von 0,01 bis 5,0 Gew.-%, bevorzugt 0,05 bis 2,0 und besonders bevorzugt 0,1 bis 1,0 Gew.-%, jeweils bezogen auf die gesamte kosmetische oder pharmazeutische Zusammensetzung, eingesetzt.
Erfindungsgemäß wird 7-Acetyl-1,1,3,4,4,6-hexamethyltetralin in Mengen von 0,001 bis 1,0 Gew.-%, bevorzugt 0,005 bis 0,5 und besonders bevorzugt 0,01 bis 0,2 Gew.-%, jeweils bezogen auf die gesamte kosmetische oder pharmazeutische Zusammensetzung, eingesetzt.

Nach einer weiteren bevorzugten Ausführungsform sind die erfindungsgemäß verwendeten Pflanzenextrakte ausgewählt aus den Extrakten aus Melisse, Grünem Tee (Camellia sinensis), Mate (Ilex paraguayensis), Japanischem Tee (Camellia japanensis), aus den Früchten (Beeren) der Fächerpalme oder Sägepalme (Saw Palmetto, Serenoa repens), aus Gingko biloba, Yuzu-Öl, aus Citrusfruchtsamen, aus den Früchten (Beeren) von Phyllanthus emblica und aus den Blättern des Olivenbaumes (Olea europaea). Besonders bevorzugt sind die Extrakte aus Grünem Tee (Camellia sinensis), Yuzu-Öl und aus Citrusfruchtsamen, ganz besonders bevorzugt sind Citrusfruchtsamen-Extrakte. Die Citrusfruchtsamen stammen dabei von allen Citrusarten, bevorzugt von Citrus sinensis, C. paradisi, C. aurantium, C. aurantifolia, C. reticulata, C. grandis, C. limonia und C. medica, besonders bevorzugt von C. aurantium, C. sinensis, C. paradisi, C. limonia und C. reticulata.
Erfindungsgemäß besonders bevorzugt verwendet werden Citrusfruchtsamen-Extrakte, die durch einen Druckaufschluss von etwa 12 - 72 Stunden, bevorzugt etwa 48 Stunden Dauer in Glycerin bei Temperaturen von etwa 50 - 150°C und anschließendem Zentrifugieren und Filtrieren erhältlich sind.

Gemäß einer besonders bevorzugten Ausführungsform sind die erfindungsgemäß verwendeten Pflanzenextrakte ausgewählt aus den Extrakten aus Meeresalgen, Apfelkernen, Pinienrinde (Pinus Pinaster), Trauben, Rotwein, Rosmarin, Hyssop (Ysop), Gewürznelke und Weidenröschen (Epilobium angustifolium, Willowherb).

Erfindungsgemäß werden die Pflanzenextrakte in Mengen von 0,001 bis 20 Gew.-%, bevorzugt 0,005 bis 10 und besonders bevorzugt 0,01 bis 5 Gew.-% Aktivsubstanz, jeweils bezogen auf die gesamte kosmetische oder pharmazeutische Zusammensetzung, eingesetzt.

In einer weiteren bevorzugten Ausführungsform sind die erfindungsgemäß verwendeten Riechstoffe ausgewählt aus dem Rohstoff Protectate HR, einem Riechstoffgemisch erhältlich von Symrise, vormals Haarmann und Reimer. Protectate HR wird erfindungsgemäß als Steroidsulfatase-Inhibitor in Mengen von 0,001 bis 5 Gew.-%, bevorzugt 0,01 bis 2 Gew.-% und besonders bevorzugt 0,1 bis 1 Gew.-%, jeweils bezogen auf die gesamte kosmetische Zusammensetzung, eingesetzt.

Da die erfindungsgemäß geeigneten Pflanzenextrakte Flavonoide enthalten, sind auch Flavonoide explizit als erfindungsgemäß geeignete und bevorzugte Steroidsulfatase-Inhibitoren offenbart.
Die erfindungsgemäß bevorzugten Flavonoide umfassen die Glycoside der Flavone, der Flavanone, der 3-Hydroxyflavone (Flavonole), der Aurone und der Isoflavone. Besonders bevorzugte Flavonoide sind ausgewählt aus α-Glucosylrutin, α-Glucosylmyricetin, α-Glucosylisoquercetin, α-Glucosylquercetin, Naringin (Aurantiin, Naringenin-7-rhamnoglucosid), Hesperidin (3',5,7-Trihydroxy-4'-methoxyflavanon-7-rhamnoglucosid, Hesperitin-7-O-rhamnoglucosid), Neohesperidin, Rutin (3,3',4',5,7-Pentahydroxyflavon-3-rhamnoglucosid, Quercetin-3-rhamnoglucosid), Troxerutin (3,5-Dihydroxy-3',4',7-tris(2-hydroxyethoxy)-flavon-3-(6-O-(6-deoxy-α-L-mannopyranosyl)-β-D-glucopyranosid)), Monoxerutin (3,3',4',5-Tetrahydroxy-7-(2-hydroxyethoxy)-flavon-3-(6-O-(6-deoxy-α-Lmannopyranosyl)-β-D-glucopyranosid)), Diosmin (3',4',7-Trihydroxy-5-methoxyflavanon-7-rhamnoglucosid), Apigenin-7-glycosid und Eriodictin.
Ebenfalls bevorzugt sind die aus zwei Flavonoideinheiten aufgebauten Biflavonoide, die z. B. in Gingko-Arten vorkommen. Weitere bevorzugte Flavonoide sind die Chalkone, vor allem Phlorizin und Neohesperidindihydrochalkon.
Erfindungsgemäß werden die Flavonoide in Mengen von 0,001 bis 1 Gew.-%, bevorzugt 0,005 bis 0,5 Gew.-% und besonders bevorzugt 0,01 bis 0,4 Gew.-%, jeweils bezogen auf die Flavonoidaktivsubstanz in der gesamten kosmetischen Zusammensetzung, eingesetzt.

In einer besonders bevorzugten Ausführungsform sind die erfindungsgemäß verwendeten Steroidsulfatase-Inhibitoren ausgewählt aus Isoflavonoiden, wobei hierzu die Isoflavone und die Isoflavon-Glycoside gezählt werden.
Unter Isoflavonen sind im Sinne der vorliegenden Erfindung Stoffe zu verstehen, die Hydrierungs-, Oxidations- oder Substitutionsprodukte des 3-Phenyl-4H-1-benzopyrans darstellen, wobei eine Hydrierung in der 2,3-Stellung des Kohlenstoffgerüsts vorliegen kann, eine Oxidation unter Ausbildung einer Carbonylgruppe in der 4-Stellung vorliegen kann, und unter Substitution der Ersatz eines oder mehrerer Wasserstoffatome durch Hydroxy- oder Methoxy-Gruppen zu verstehen ist. Zu den erfindungsgemäßen Isoflavonen zählen beispielsweise Daidzein, Genistein, Prunetin, Biochanin, Orobol, Santal, Pratensein, Irigenin, Glycitein, Biochanin A und Formononetin. Als Isoflavone bevorzugt sind Daidzein, Genistein, Glycitein und Formononetin.

In den erfindungsgemäß verwendeten Isoflavon-Glycosiden sind die Isoflavone über mindestens eine Hydroxygruppe mit mindestens einem Zucker glycosidisch verknüpft. Als Zucker kommen Mono- oder Oligosaccharide, insbesondere D-Glucose, D-Galactose, D-Glucuronsäure, D-Galacturonsäure, D-Xylose, D-Apiose, L-Rhamnose, L-Arabinose und Rutinose in Betracht. Bevorzugte Beispiele für die erfindungsgemäß verwendeten Isoflavon-Glycoside sind Daidzin und Genistin.

Erfindungsgemäß weiterhin bevorzugt ist es, wenn die Isoflavone und/oder deren Glycoside als Bestandteile eines aus einer Pflanze gewonnenen Substanzgemisches, insbesondere eines pflanzlichen Extraktes, in den Zubereitungen enthalten sind. Solche pflanzlichen Substanzgemische können in dem Fachmann geläufiger Weise beispielsweise durch Auspressen oder Extrahieren aus Pflanzen wie Soja, Rotklee oder Kichererbsen gewonnen werden. Besonders bevorzugt werden in den erfindungsgemäßen Zubereitungen Isoflavone oder Isoflavon-Glycoside in Form von aus Soja gewonnenen Extrakten eingesetzt, wie sie beispielsweise unter der Produktbezeichnung Soy Protein Isolate SPI (Protein Technology International, St. Louis) oder Soy Phytochemicals Concentrate SPC (Archer Daniels Midland, Decatur) im Handel erhältlich sind.

Erfindungsgemäß werden die Isoflavonoide in Mengen von 0,001 bis 1 Gew.-%, bevorzugt 0,005 bis 0,5 Gew.-% und besonders bevorzugt 0,01 bis 0,4 Gew.-%, jeweils bezogen auf die Isoflavonoid-Aktivsubstanz in der gesamten kosmetischen Zusammensetzung, eingesetzt.

In einer weiteren besonders bevorzugten Ausführungsform sind die erfindungsgemäß verwendeten Steroidsulfatase-Inhibitoren ausgewählt aus 6,7-disubstituierten 2,2-Dialkylchromanen oder -chromenen der allgemeinen Formeln (I) oder (II), 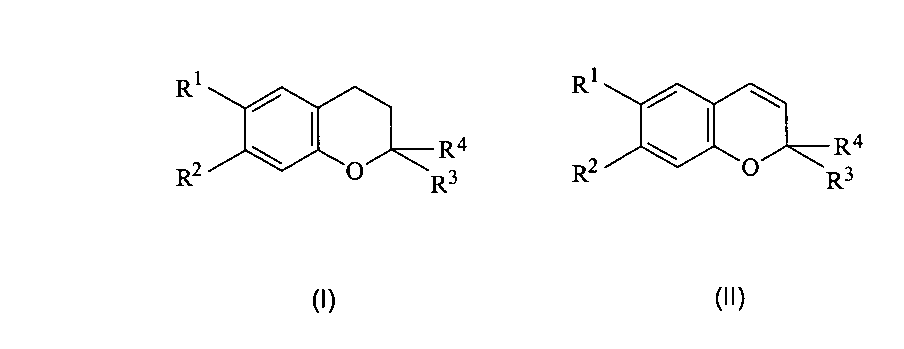 wobei R¹ und R² unabhängig voneinander eine OH-Gruppe, eine Methoxy-Gruppe oder eine CF₃CH₂O-Gruppe und R³ und R⁴ unabhängig voneinander eine C₁-C₄-Alkylgruppe darstellen.

Die Substituenten R¹ und R² sind unabhängig voneinander ausgewählt aus einer OH-Gruppe, einer Methoxy-Gruppe und einer CF₃CH₂O-Gruppe. In einer bevorzugten Ausführungsform sind R¹ und R² unabhängig voneinander ausgewählt aus einer OH-Gruppe und einer Methoxy-Gruppe. In einer besonders bevorzugten Ausführungsform stellen R¹ eine OH-Gruppe und R² eine Methoxy-Gruppe dar.

Die Substituenten R³ und R⁴ stellen unabhängig voneinander eine C₁-C₄-Alkylgruppe dar. Unter C₁-C₄-Alkylgruppe ist dabei erfindungsgemäß eine Methyl-, Ethyl-, n-Propyl-, Isopropyl-, n-Butyl-, Isobutyl- beziehungsweise 2-Methylpropyl-, sec-Butyl- beziehungsweise 1-Methylpropyl- oder eine tert.-Butyl-Gruppe zu verstehen. In einer bevorzugten Ausführungsform sind R³ und R⁴ unabhängig voneinander ausgewählt aus einer Methyl-, Ethyl-, n-Propyl-, Isopropyl- und einer n-Butyl-Gruppe. Besonders bevorzugt stellen R³ und R⁴ unabhängig voneinander eine Methyl- oder Ethylgruppe dar. Außerordentlich bevorzugt ist, dass R³ und R⁴ identisch sind.

Erfindungsgemäß bevorzugt verwendet werden die 6,7-disubstituierten 2,2-Dialkylchromane. Ein erfindungsgemäß außerordentlich bevorzugt verwendeter Wirkstoff ist 2,2-Dimethyl-6-hydroxy-7-methoxy-chroman mit der systematischen Bezeichnung 3,4-Dihydro-7-methoxy-2,2-dimethyl-2H-1-benzopyran-6-ol und der INCl-Bezeichnung Dimethylmethoxy Chromanol. Die Substanz ist unter dem Handelsnamen Lipochroman-6 von der Firma Lipotec S.A. erhältlich.

Die 6,7-disubstituierten 2,2-Dialkylchromane oder -chromene der allgemeinen Formeln (I) oder (II) werden erfindungsgemäß in Mengen von 0,001 - 5 Gew.-%, bevorzugt 0,005 - 2 Gew.-% und besonders bevorzugt 0,01 - 1 Gew.-%, jeweils bezogen auf die gesamte kosmetische oder pharmazeutische Zusammensetzung, eingesetzt.

In einer weiteren bevorzugten Ausführungsform sind die erfindungsgemäß verwendeten funktionellen C₂-C₁₂-Carbonsäuren ausgewählt aus C₂-C₁₂-Hydroxycarbonsäuren, C₃-C₁₂-Ketocarbonsäuren, C₂-C₅-Aminosäuren und deren N-C₂-C₁₂-Acylderivaten sowie den physiologisch verträglichen Metallsalzen dieser Säuren.
Die physiologisch verträglichen Metallsalze sind ausgewählt aus den Alkalimetall-, Magnesium-, Calcium-, Aluminium-, Zink- und Mangan-Salzen. Bevorzugt sind die Natrium-, Kalium-, Magnesium-, Aluminium-, Zink- und Mangan-Salze.
Erfindungsgemäß werden die funktionellen C₂-C₁₂-Carbonsäuren und ihre physiologisch verträglichen Metallsalze in Mengen von 0,001 bis 10 Gew.-%, bevorzugt 0,005 bis 5 Gew.-% und besonders bevorzugt 0,01 bis 2 Gew.-%, jeweils bezogen auf die gesamte kosmetische oder pharmazeutische Zusammensetzung, eingesetzt.

Erfindungsgemäß bevorzugt verwendete C₂-C₁₂-Hydroxycarbonsäuren und ihre physiologisch verträglichen Metallsalze sind ausgewählt aus den alpha-Monohydroxy-C₃-C₁₂-Carbonsäuren und ihren Salzen, zum Beispiel Milchsäure, Aluminiumlactat, Zinklactat, alpha-Hydroxycaprinsäure, alpha-Hydroxycaprylsäure und alpha-Hydroxylaurinsäure, sowie den Polyhydroxy-C₃-C₈-Carbonsäuren, zum Beispiel D-Gluconsäure, Aluminiumgluconat, Zinkgluconat, Mangangluconat, D-Glucoheptonsäure und Magnesiumglucoheptonat.

Erfindungsgemäß bevorzugt verwendete C₃-C₁₂-Ketocarbonsäuren sind beispielsweise Brenztraubensäure, Acetessigsäure und Lävulinsäure (4-Oxopentansäure). Besonders bevorzugt ist Lävulinsäure.

Erfindungsgemäß bevorzugt verwendete C₂-C₅-Aminosäuren, deren N-C₂-C₁₂-Acylderivate sowie die physiologisch verträglichen Metallsalze der genannten Verbindungen sind beispielsweise Glycin, Aluminiumglycinat, Alanin, Glutaminsäure, Pyroglutaminsäure, Zinkpyroglutamat, Natriumoctanoylglutamat, Natriumdecanoylglutamat, Natriumlauroylglutamat, Natriummyristoylglutamat, Natriumcetoylglutamat und Natriumstearoylglutamat. Besonders bevorzugt sind Aluminiumglycinat, Zinkpyroglutamat und Natriumlauroylglutamat.

Erfindungsgemäß bevorzugt verwendete Derivate von C₃-C₉-Polyolen sind ausgewählt aus Ascorbinsäure, Monoalkyl-C₃-C₉-Polyolethern, Ethern und Estern von gewünschtenfalls alkylierter Glucose sowie den Tetraestern von Pentaerythrit mit einen substituierten Steroidrest enthaltenden C₂-C₄-Carbonsäuren. Bevorzugt verwendete Monoalkyl-C₃-C₉-Polyolether sind zum Beispiel Monoalkylglycerinether, besonders bevorzugt Hexylglycerinether, 2-Ethylhexylglycerinether, 2-Ethyloctylglycerinether und 2-Ethyldecylglycerinether. Ein bevorzugt verwendeter Glucoseether ist Ascorbylglucosid. Bevorzugt verwendete Glucoseester sind die Ester von hydroxysubstituierten Bi- oder Tricarbonsäuren mit alkylierter Glucose der allgemeinen Formel (III), 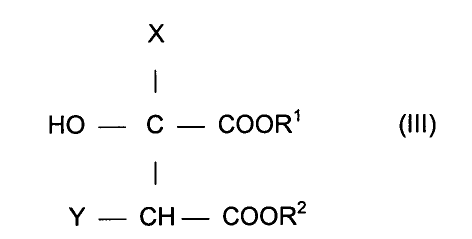 in der X ein Wasserstoff-Atom oder eine -CH₂COOR-Gruppe ist, Y ein Wasserstoff-Atom oder -OH-Gruppe ist unter der Bedingung, dass Y ein Wasserstoff-Atom ist, wenn X -CH₂COOR ist, R, R¹ und R² unabhängig voneinander ein Wasserstoffatom, ein Alkali- oder Erdalkalimetallkation, eine Ammoniumgruppe, das Kation einer ammonium-organischen Base oder einen Rest Z bedeuten, wobei Z einen mit einem C₆-C₁₈-Alkanol oder mit technischen Mischungen von C₆-C₁₈-Alkanolen veretherten Glucose- oder Oligoglucoserest darstellt, mit der Bedingung, dass mindestens eine der Gruppen R, R¹ oder R² ein Rest Z ist. Solche Verbindungen sind z. B. in der Druckschrift EP 258 814 B1 beschrieben. Besonders bevorzugt verwendete Verbindungen der Formel (III) sind Dinatrium-Kokosalkylpolyglucosidcitrat (Disodium Cocopolyglucose Citrate, als Handelsprodukt Eucarol® AGE EC von der Firma Cesalpinia Chemicals erhältlich) und Natrium-Kokosalkylpolyglucosidtartrat (Sodium Cocopolyglucose Tartrate, als Handelsprodukt Eucarol® AGE ET von der Firma Cesalpinia Chemicals erhältlich). Bevorzugt verwendete Tetraester von Pentaerythrit mit einen substituierten Steroidrest enthaltenden C₂-C₄-Carbonsäuren sind abgeleitet von Essigsäure, die am C2-Atom einen substituierten Steroidrest aufweist, Propionsäure, die am C3-Atom einen substituierten Steroidrest aufweist und Buttersäure, die am C4-Atom einen substituierten Steroidrest aufweist. Die substituierten Steroidreste weisen bevorzugt in 4-Stellung eine Hydroxylgruppe sowie in 3- und/oder 5-Stellung eine C₁-C₄-Alkylgruppe auf. Diese C₁-C₄-Alkylgruppen sind ausgewählt aus Methyl-, Ethyl-, n-Propyl-, Isopropyl, n-Butyl-, sec-Butyl- und tert-Butyl-Gruppen. Eine besonders bevorzugt verwendete Verbindung dieses Typs ist Pentaerythrittetrakis[3(3,5-di-tert.-butyl-4-hydroxyphenyl)propionat], als Handelsprodukt Tinogard® TT DD von Ciba erhältlich.
Erfindungsgemäß werden die Derivate von C₃-C₉-Polyolen in Mengen von 0,01 bis 15 Gew.-%, bevorzugt 0,05 bis 10 Gew.-% und besonders bevorzugt 0,1 bis 3 Gew.-%, jeweils bezogen auf die gesamte kosmetische oder pharmazeutische Zusammensetzung, eingesetzt.

Erfindungsgemäß bevorzugt verwendete Phenolderivate sind ausgewählt aus Phenoxyethanol, Salicylsäure, Rosmarinsäure, Kaffeesäure, Tocopherolen und Tocopherylestern, insbesondere α-Tocopherol, Tocopherylacetat, Tocopherylnicotinat, Tocopherylphosphat und Tocopherylsuccinat, Benzotriazolyl-substituierten 4-Phenolsulfonsäuren und deren Salzen sowie Polyphenolen. Unter Polyphenolen sind erfindungsgemäß aromatische Verbindungen zu verstehen, die mindestens zwei phenolische Hydroxy-Gruppen im Molekül enthalten. Hierzu zählen die drei Dihydroxybenzole Brenzcatechin, Resorcin und Hydrochinon, weiterhin Phloroglucin, Pyrogallol und Hexahydroxybenzol. In der Natur treten freie und veretherte Polyphenole beispielsweise in Blütenfarbstoffen (Anthocyanidine, Flavone), in Gerbstoffen (Catechine, Tannine), als Flechtenoder Farn-Inhaltsstoffe (Usninsäure, Acylpolyphenole), in Ligninen und als Gallussäure-Derivate auf. Bevorzugte Polyphenole sind Flavone, Catechine, Usninsäure, und als Tannine die Derivate der Gallussäure, Digallussäure und Digalloylgallussäure. Besonders bevorzugte Polyphenole sind die monomeren Catechine, das heißt die Derivate der Flavan-3-ole, und Leukoanthocyanidine, das heißt die Derivate der Leukoanthocyanidine, die bevorzugt in 5,7,3',4',5'-Stellung phenolische Hydroxygruppen tragen, bevorzugt Epicatechin und Epigallocatechin, sowie die daraus durch Selbstkondensation entstehenden Gerbstoffe. Solche Gerbstoffe werden bevorzugt nicht in isolierter Reinsubstanz, sondern als Extrakte gerbstoffreicher Pflanzenteile eingesetzt, z. B. Extrakte von Catechu, Quebracho, Pinienrinde und Eichenrinde sowie anderer Baumrinden, Blättern von Grünem Tee (camellia sinensis) und Mate. Ebenfalls besonders bevorzugt sind die Tannine.

Ein besonders bevorzugter Polyphenol-reicher kosmetischer Wirkstoff ist das Handelsprodukt Sepivinol R, ein Extrakt aus Rotwein, erhältlich von der Firma Seppic.
Erfindungsgemäß werden die Polyphenole in Mengen von 0,001 bis 10 Gew.-%, bevorzugt 0,005 bis 5 Gew.-% und besonders bevorzugt 0,08 bis 3 Gew.-%, jeweils bezogen auf die gesamte kosmetische oder pharmazeutische Zusammensetzung, eingesetzt.

Die bevorzugt verwendeten Benzotriazolyl-substituierten 4-Phenolsulfonsäuren und ihre Salze enthalten den Benzotriazolyl-Rest in 3-Position. Besonders bevorzugt werden solche Verbindungen verwendet, die zusätzlich in der 5-Position eine C₁-C₄-Alkylgruppe aufweisen. Die C₁-C₄-Alkylgruppen sind dabei ausgewählt aus Methyl-, Ethyl-, n-Propyl-, Isopropyl-, n-Butyl-, sec-Butyl- und tert-Butyl-Gruppen. Erfindungsgemäß geeignete Salze der 4-Phenolsulfonsäuren sind alle mit physiologisch verträglichen Kationen, beispielsweise Natrium, Kalium und Ammonium. Besonders bevorzugt verwendet wird das 3-Benzotriazolyl-5-secbutyl-4-phenolsulfonsäure-natriumsalz, als Handelsprodukte Tinogard™ HS (Reinsubstanz) oder Cibafast™ H (Compound mit Buteth-3 und Tributylcitrat) von Ciba erhältlich.

Besonders bevorzugt sind außerdem Phenoxyethanol, Tocopherylacetat und Rosmarinsäure.
Erfindungsgemäß werden die Phenolderivate in Mengen von 0,001 bis 10 Gew.-%, bevorzugt 0,005 bis 5 Gew.-% und besonders bevorzugt 0,08 bis 3 Gew.-%, jeweils bezogen auf die gesamte kosmetische oder pharmazeutische Zusammensetzung, eingesetzt.

Ein erfindungsgemäß besonders bevorzugt verwendetes Tropolonderivat ist Hinokitiol. Erfindungsgemäß werden die Tropolonderivate in Mengen von 0,0001 bis 5 Gew.-%, bevorzugt 0,0005 bis 3 Gew.-% und besonders bevorzugt 0,005 bis 0,5 Gew.-%, jeweils bezogen auf die gesamte kosmetische oder pharmazeutische Zusammensetzung, eingesetzt.

Erfindungsgemäß bevorzugt verwendete Ester der Schwefelsäure mit alkoxylierten C₈-C₁₈-Fettalkoholen und deren physiologisch verträglichen Metallsalze sind aus Cocosalkohol erhältlich und haben einen Ethoxylierungsgrad n von 1 bis 20, bevorzugt 1 bis 15 und besonders bevorzugt 2 bis 10. Besonders bevorzugt sind die Zinksalze dieser Schwefelsäureester. Erfindungsgemäß besonders bevorzugt ist Zinkcocoylethersulfat mit einem Ethoxylierungsgrad n von 1 bis 20, wobei Ethoxylierungsgrade von n = 2, 3, 4, 5, 6, 7, 8, 9 und 10 ganz besonders bevorzugt sind unter besonderer Bevorzugung des Wertes 3.

Ein weiterer Gegenstand der vorliegenden Erfindung sind kosmetische Zubereitungen, insbesondere Haarbehandlungsmittel, zur Verminderung von Haarausfalls, die mindestens einen Ester der Schwefelsäure mit alkoxylierten C₈-C₁₈-Fettalkoholen und/oder deren physiologisch verträgliche Metallsalze mit einem Alkoxylierungsgrad n von 1 - 30 enthalten. In einer bevorzugten Ausführungsform enthalten die erfindungsgemäßen Haarbehandlungsmittel Zinkcocoylethersulfat mit einem Ethoxylierungsgrad n von 1 bis 20, wobei Ethoxylierungsgrade von n = 2, 3, 4, 5, 6, 7, 8, 9 und 10 ganz besonders bevorzugt sind unter besonderer Bevorzugung des Wertes 3.

Erfindungsgemäß werden die Ester der Schwefelsäure mit alkoxylierten C₈-C₁₈-Fettalkoholen und/oder deren physiologisch verträglichen Metallsalze in Mengen von 0,01 bis 10 Gew.-%, bevorzugt 0,03 bis 5 Gew.-% und besonders bevorzugt 0,05 bis 1 Gew.-%, jeweils bezogen auf die gesamte kosmetische oder pharmazeutische Zusammensetzung, eingesetzt.

Erfindungsgemäß bevorzugt verwendete Ester der Phosphorsäure und der Triphosphorsäure mit ein- bis sechswertigen Hydroxyverbindungen sind ausgewählt aus den Phosphaten und Triphosphaten von alkoxylierten C₈-C₂₀-Alkanolen, Inositolen und N-Glykosiden. Besonders bevorzugte Phosphate von alkoxylierten C₈-C₂₀-Alkanolen enthalten sowohl n Ethylenoxid- als auch m Propylenoxid-Einheiten mit n = 2 bis 20 und m = 2 bis 10, die an bevorzugt lineare C₈-C₂₀-Alkanole wie Caprylalkohol, Caprinalalkohol, Laurylalkohol, Myristylalkohol, Cetylalkohol, Stearylalkohol und Arachidylalkohol, bevorzugt C₁₂-C₁₈-Alkanole und besonders bevorzugt C₁₆-C₁₈-Alkanole und deren technische Gemische addiert sind. Ein besonders bevorzugtes Beispiel hierfür ist PPG-5-Ceteth-10 Phosphat.
Ein erfindungsgemäß besonders geeignetes Inositolphosphat ist *myo* -Inosithexaphosphat (Phytinsäure).
Ein erfindungsgemäß besonders geeignetes N-Glykosid-Triphosphat ist Adenosintriphosphat (ATP).
Erfindungsgemäß werden die Ester der Phosphorsäure und der Triphosphorsäure mit ein- bis sechswertigen Hydroxyverbindungen in Mengen von 0,01 bis 10 Gew.-%, bevorzugt 0,05 bis 5 Gew.-% und besonders bevorzugt 0,25 bis 2 Gew.-%, jeweils bezogen auf die gesamte kosmetische oder pharmazeutische Zusammensetzung, eingesetzt.

Gemäß einer weiteren bevorzugten Ausführungsform der Erfindung werden als Steroidsulfatase-Inhibitoren Kieselsäureester verwendet, wobei die Kieselsäureester ausgewählt sind aus Verbindungen der Formeln (IV), (V), (VI), (VII) und (VIII), 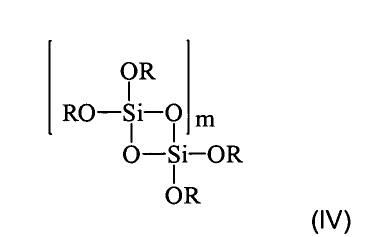 und 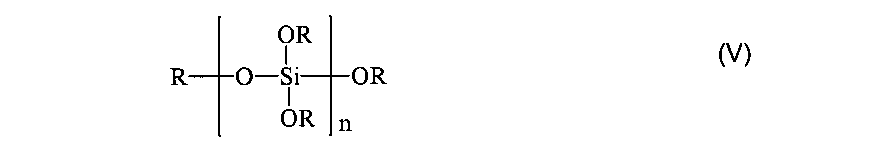 und 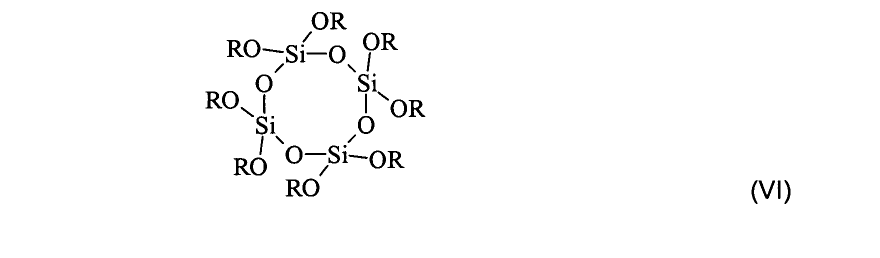 und 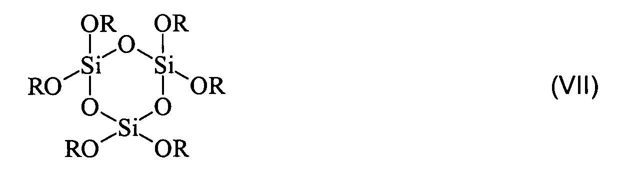 und 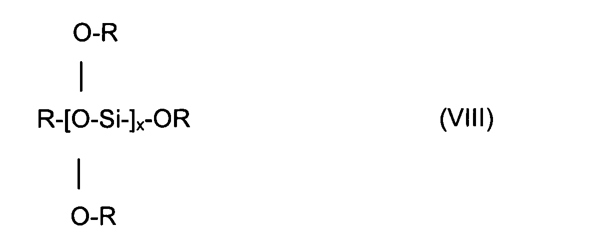 wobei alle R unabhängig voneinander ausgewählt sind aus der Gruppe, die ein Wasserstoffatom, die geradkettigen oder verzweigten, gesättigten oder ungesättigten, substituierten oder unsubstituierten C₁₋₆-Kohlenwasserstoffreste und den 2-Phenoxyethyl-Rest enthält, wobei mindestens ein R verschieden von einem Wasserstoffatom sein muss, und m Werte aus dem Bereich 1 bis 20 und n Werte aus dem Bereich 2 bis 100 und x Werte aus dem Bereich 2 bis 20 annimmt, sowie Mischungen hiervon.
Oligokieselsäureester niederer Alkohole sind kommerziell erhältlich, wobei üblicherweise Methanol, Ethanol, n-Propanol, iso-Propanol, n-Butanol, iso-Butanol und tert.-Butanol zur Veresterung eingesetzt wurden. Möglich ist die Verwendung von Kieselsäureestermischungen, in denen ein Teil der Reste R ausgewählt ist aus der Gruppe Methyl, Ethyl, n-Propyl, iso-Propyl, n-Butyl, iso-Butyl und tert.-Butyl. Den Einbau des 2-Phenoxyethyl-Restes erzielt man durch Umesterung der Kieselsäureester niederer Alkohole mit 2-Phenoxyethanol.
Die Oligomerisierungsgrade "n" der erfindungsgemäß verwendeten Kieselsäureester liegen zwischen 2 und 20. In bevorzugt verwendeten Verbindungen nimmt n Werte zwischen 2 und 15, vorzugsweise zwischen 2 und 12 und insbesondere zwischen 3 und 10 unter besonderer Bevorzugung der Werte 4, 5, 6, 7 und 8, an.
Erfindungsgemäß werden die Kieselsäureester in Mengen von 0,001 bis 5 Gew.-%, bevorzugt 0,005 bis 3 Gew.-% und besonders bevorzugt 0,01 bis 2 Gew.-%, jeweils bezogen auf die gesamte kosmetische oder pharmazeutische Zusammensetzung, eingesetzt.
Besonders bevorzugt werden die Kieselsäureester in im wesentlichen wasserfreien Zusammensetzungen eingesetzt, die maximal 5 Gew.-%, bevorzugt maximal 1 Gew.-% Wasser enthalten.

Nach einer weiteren bevorzugten Ausführungsform der Erfindung werden als Steroidsulfatase-Inhibitoren Mycosporin-ähnliche Aminosäuren (Mycosporin-like Amino Acids, MAA) verwendet, die aus marinen Organismen erhältlich sind. Einen Überblick über MAA liefern beispielsweise D. Karentz et al., Marine Biology 108, 157 - 166, 1991. Mycosporin ist der generische Name für wasserlösliche, UVabsorbierende Stoffwechselprodukte von beispielsweise Pilzen, Algen und Cyanobakterien, deren Moleküle ein Cyclohexenon-Chromophor enthalten, das mit der Aminogruppe einer Aminosäure oder eines Aminoalkohols konjugiert ist. Die Mycosporin-ähnlichen Aminosäuren, die in marinen Organismen enthalten sind, sind Iminocarbonyl-Derivate des Mycosporin-Cyclohexenon-Chromophors.
Erfindungsgemäß werden die MAAs in Mengen von 0,001 bis 1 Gew.-%, bevorzugt 0,01 bis 0,5 Gew.-% und besonders bevorzugt 0,02 bis 0,5 Gew.-%, jeweils bezogen auf die gesamte kosmetische Zusammensetzung, eingesetzt.
MAA sind hydrolyseempfindlich, daher werden sie, ähnlich wie die erfindungsgemäß verwendeten Kieselsäureester, bevorzugt in wasserfreien Zusammensetzungen eingesetzt.

Erfindungsgemäß bevorzugt verwendete quaternäre Siliconverbindungen sind ausgewählt aus Amodimethiconen, Trimethylsilylamodimethiconen und Quaternium-80, beispielsweise die im Handel erhältlichen Produkte Abil®-Quat 3474, Abil®-Quat 3270 und Abil®-Quat 3272 ((Kokosamidopropoxy)propylacetat-Ammonium-modifizierte diquaternäre Polydimethylsiloxane, INCl-Bezeichnung: Quaternium-80; Hersteller: Th. Goldschmidt), Q2-7224 (Hersteller: Dow Corning; ein stabilisiertes Trimethylsilylamodimethicon), Dow Corning® 929 Emulsion (enthaltend ein hydroxyl-amino-modifiziertes Silicon, das auch als Amodimethicone bezeichnet wird), SM-2059 (Hersteller: General Electric) sowie SLM-55017 (Wacker).
Erfindungsgemäß werden die quaternären Siliconverbindungen in Mengen von 0,1 bis 10 Gew.-%, bevorzugt 0,5 bis 5 Gew.-% und besonders bevorzugt 1,0 bis 5 Gew.-%, jeweils bezogen auf den Anteil des Handelsproduktes an der gesamten kosmetischen Zusammensetzung, eingesetzt.

Die kosmetischen Zubereitungen können beispielsweise zur Herstellung von Haarshampoos, Haarspülungen, Haarlotionen, Haarkuren oder -fluids, Festigern oder Lotionen dienen, welche ihrerseits als weitere Hilfs- und Zusatzstoffe milde Tenside, Ölkörper, Emulgatoren, Überfettungsmittel, Perlglanzwachse, Stabilisatoren, Konsistenzgeber, Verdickungsmittel, Siliconverbindungen, biogene Wirkstoffe, Deowirkstoffe, Antischuppenmittel, Filmbildner, Konservierungsmittel, Hydrotrope, Solubilisatoren, UV-Lichtschutz-faktoren, Antioxidantien, Insektenrepellentien, Selbstbräuner, Parfümöle, Farbstoffe und dergleichen enthalten können

Typische Beispiele für geeignete milde, d.h. besonders hautverträgliche Tenside sind Fettalkoholpoly-glycolethersulfate, Monoglyceridsulfate, Mono- und/oder Dialkylsulfosuccinate, Fettsäureisethionate, Fettsäuresarcosinate, Fettsäuretauride, Fettsäureglutamate, Ethercarbonsäuren, Alkyloligoglucoside, Fettsäureglucamide, Alkylamidobetaine und/oder Proteinfettsäurekondensate, letztere vorzugsweise auf Basis von Weizenproteinen.

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, Ester von linearen C₆-C₂₂-Fettsäuren mit verzweigten Alkoholen, insbesondere 2-Ethylhexanol, Ester von Hydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoe-säure, Ester von C₂-C₁₂-Dicarbonsäuren mit linearen oder verzweigten Alkoholen mit 1 bis 22 Kohlen-stoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalko-holcarbonate, Guerbetcarbonate, Ester der Benzoesäure mit linearen und/oder verzweigten C₆-C₂₂-Alkoholen (z.B. Finsolv® TN), lineare oder verzweigte, symmetrische oder unsymmetrische Dialkyl-ether mit 6 bis 22 Kohlenstoffatomen pro Alkylgruppe, Ringöffnungsprodukte von epoxidierten Fettsäureestern mit Polyolen, Siliconöle und/oder aliphatische bzw. naphthenische Kohlenwasserstoffe in Betracht.

Als Emulgatoren kommen beispielsweise nichtionogene Tenside aus mindestens einer der folgenden Gruppen in Frage:
(1) Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) C_{12/18}-Fettsäuremono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(4) Alkylmono- und -oligoglycoside mit 8 bis 22 Kohlenstoffatomen im Alkylrest und deren ethoxylierte Analoga;
(5) Anlagerungsprodukte von 15 bis 60 Mol Ethylenoxid an Ricinusöl und/oder gehärtetes Ricinusöl;
(6) Polyol- und insbesondere Polyglycerinester, wie z.B. Polyglycerinpolyricinoleat, Polyglycerinpoly-12-hydroxystearat oder Polyglycerindimerat. Ebenfalls geeignet sind Gemische von Verbindungen aus mehreren dieser Substanzklassen;
(7) Anlagerungsprodukte von 2 bis 15 Mol Ethylenoxid an Ricinusöl und/oder gehärtetes Ricinusöl;
(8) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipenta-erythrit, Zuckeralkohole (z.B. Sorbit), Alkylglucoside (z.B. Methylglucosid, Butylglucosid, Lauryl-glucosid) sowie Polyglucoside (z.B. Cellulose);
(9) Mono-, Di- und Trialkylphosphate sowie Mono-, Di- und/oder Tri-PEGalkylphosphate und deren Salze;
(10) Wollwachsalkohole;
(11) Polysiloxan-Polyalkyl-Polyether-Copolymere bzw. entsprechende Derivate;
(12) Mischester aus Pentaerythrit, Fettsäuren, Citronensäure und Fettalkohol gemäß DE-PS 1165574 und/oder Mischester von Fettsäuren mit 6 bis 22 Kohlenstoffatomen, Methylglucose und Polyolen, vorzugsweise Glycerin oder Polyglycerin sowie
(13) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologen-gemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus DE-PS 2024051 als Rückfettungsmittel für kosmetische Zubereitungen bekannt.

C_{8/18}-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosac-chariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylamino-propyl-N,N-dimethylammoniumglycinate, beispielsweise das Kokosacylaminopropyldimethylammonium-glycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder - Acylgruppe im Molekül mindestens eine freie Aminogruppe und mindestens eine - COOH- oder -SO₃H-Gruppe enthalten und zur Ausbildung innerer Salze befähigt sind. Beispiele für geeignete ampho-lytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkyl-gruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methyl-quaternierte Difettsäuretriethanolaminester-Salze, besonders bevorzugt sind.

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als Perlglanzwachse kommen beispielsweise in Frage: Alkylenglycolester, speziell Ethylenglycol-distearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe min-destens 24 Kohlenstoffatome aufweisen, speziell Lauron und Distearylether; Fettsäuren wie Stea-rinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxy-fettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B.
Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologen-verteilung oder Alkyloligoglucoside sowie Elektrolyte wie Kochsalz und Ammoniumchlorid.

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vor-liegen können. Eine detaillierte Übersicht über geeignete flüchtige Silicone findet sich zudem von Todd et al. in Cosm.Toil. 91, 27 (1976).
Typische Beispiele für Fette sind Glyceride, als Wachse kommen u.a. Bienenwachs, Carnaubawachs, Candelillawachs, Montanwachs, Paraffinwachs oder Mikrowachse gegebenenfalls in Kombination mit hydrophilen Wachsen, z.B. Cetylstearylalkohol oder Partialglyceriden in Frage. Als Stabilisatoren können Metallsalze von Fettsäuren, wie z.B. Magnesium-, Aluminium- und/oder Zinkstearat bzw. -ricinoleat eingesetzt werden.

Unter biogenen Wirkstoffen sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säu-ren, Aminosäuren, Ceramide, Pseudoceramide, essentielle Öle, Pflanzenextrakte und Vitaminkomplexe zu verstehen.

Als Deowirkstoffe kommen z.B. Antiperspirantien wie etwa Aluminiumchlorhydrate in Frage. Hierbei handelt es sich um farblose, hygroskopische Kristalle, die an der Luft leicht zerfließen und beim Eindampfen wäßriger Aluminiumchloridlösungen anfallen. Aluminiumchlorhydrat wird zur Herstellung von schweißhemmenden und desodorierenden Zubereitungen eingesetzt und wirkt wahrscheinlich über den partiellen Verschluß der Schweißdrüsen durch Eiweiß- und/oder Polysaccharidfällung [vgl. J.Soc.Cosm.Chem. 24, 281 (1973)]. Unter der Marke Locron® der Hoechst AG, Frankfurt/FRG, befindet beispielsweise sich ein Aluminiumchlorhydrat im Handel, das der Formel [Al₂(OH)₅Cl]*2,5 H₂O entspricht und dessen Einsatz besonders bevorzugt ist [vgl. J.Pharm.Pharmacol. 26, 531 (1975)]. Neben den Chlorhydraten können auch Aluminiumhydroxylactate sowie saure Aluminium/Zirko-niumsalze eingesetzt werden. Als weitere Deowirkstoffe können Esteraseinhibitoren zugesetzt werden. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT, Henkel KGaA, Düssel-dorf/FRG). Die Stoffe inhibieren die Enzymaktivität und reduzieren dadurch die Geruchsbildung. Wahr-scheinlich wird dabei durch die Spaltung des Citronensäureesters die freie Säure freigesetzt, die den pH-Wert auf der Haut soweit absenkt, daß dadurch die Enzyme inhibiert werden. Weitere Stoffe, die als Esteraseinhibitoren in Betracht kommen, sind Dicarbonsäuren und deren Ester, wie beispielsweise Glutarsäure, Glutarsäuremonoethylester, Glutarsäurediethylester, Adipinsäure, Adipinsäuremono-ethylester, Adipinsäurediethylester, Malonsäure und Malonsäurediethylester, Hydroxycarbnonsäuren und deren Ester wie beispielsweise Citronensäure, Äpfelsäure, Weinsäure oder Weinsäurediethylester. Antibakterielle Wirkstoffe, die die Keimflora beeinflussen und schweißzersetzende Bakterien abtöten bzw. in ihrem Wachstum hemmen, können ebenfalls in den Stiftzubereitungen enthalten sein. Beispiele hierfür sind Chitosan, Phenoxyethanol und Chlorhexidingluconat. Besonders wirkungsvoll hat sich auch 5-Chlor-2-(2,4-dichlorphen-oxy)-phenol erwiesen, das unter der Marke Irgasan® von der Ciba-Geigy, Basel/CH vertrieben wird.

Als Antischuppenmittel können Climbazol, Octopirox und Zinkpyrethion eingesetzt werden. Gebräuchliche Filmbildner sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen. Als Quellmittel für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in Cosm.Toil. 108, 95 (1993) entnommen werden.

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
- 3-Benzylidencampher bzw. 3-Benzylidennorcampher und dessen Derivate, z.B. 3-(4-Methylben-zyliden)campher wie in der EP-B1 0693471 beschrieben;
- 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäure-propylester, 4-Methoxyzimtsäureisoamylester 2-Cyano-3,3-phenylzimtsäure-2-ethylhexylester (Oc-tocrylene);
- Ester der Salicylsäure, vorzugsweise Salicylsäure-2-ethylhexylester, Salicylsäure-4-isopropylben-zylester, Salicylsäurehomomenthylester;
- Derivate des Benzophenons, vorzugsweise 2-Hydroxy-4-methoxybenzophenon, 2-Hydroxy-4-meth-oxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin und Octyl Tria-zon, wie in der EP-A1 0818450 beschrieben;
- Propan-1,3-dione, wie z.B. 1-(4-tert.Butylphenyl)-3-(4'methoxyphenyl)propan-1,3-dion;
- Ketotricyclo(5.2.1.0)decan-Derivate, wie in der EP-B1 0694521 beschrieben.

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- Sulfonsäurederivate von Benzophenonen, vorzugsweise 2-Hydroxy-4-methoxybenzophenon-5-sul-fonsäure und ihre Salze;
- Sulfonsäurederivate des 3-Benzylidencamphers, wie z.B. 4-(2-Oxo-3-bornylidenmethyl)benzolsul-fonsäure und 2-Methyl-5-(2-oxo-3-bornyliden)sulfonsäure und deren Salze.

Als typische UV-A-Filter kommen insbesondere Derivate des Benzoylmethans in Frage, wie beispiels-weise 1-(4'-tert.Butylphenyl)-3-(4'-methoxyphenyl)propan-1,3-dion, 4-tert.-Butyl-4'-methoxydibenzoyl-methan (Parsol 1789), oder 1-Phenyl-3-(4'-isopropylphenyl)-propan-1,3-dion. Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse Metall-oxide bzw. Salze in Frage, wie beispielsweise Titandioxid, Zinkoxid, Eisenoxid, Aluminiumoxid, Ceroxid, Zirkoniumoxid, Silicate (Talk), Bariumsulfat und Zinkstearat. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphä-rischen Gestalt abweichende Form besitzen. Weitere geeignete UV-Lichtschutzfilter sind der Übersicht von P.Finkel in SÖFW-Journal 122, 543 (1996) zu entnehmen.
Neben den beiden vorgenannten Gruppen primärer Lichtschutzstoffe können auch sekundäre Licht-schutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Auro-thioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butylund Lauryl-, Palmitoyl-, Oleyl-, γ-Linoleyl-, Cholesteryl- und Glycerylester ) sowie deren Salze, Dilaurylthiodipropionat, Distearyl-thiodipropionat, Thiodipropionsäure und deren Derivate (Ester, Ether, Peptide, Lipide, Nukleotide, Nuk-leoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phy-tinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fett-säuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-Eacetat), Vitamin A und Derivate (Vitamin-A-palmitat) sowie Koniferylbenzoat des Benzoeharzes, Rutinsäure und deren Deri-vate, α-Glycosylrutin, Ferulasäure, Furfurylidenglucitol, Carnosin, Butylhydroxytoluol, Butylhydroxy-anisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vor-zugsweise 2 bis 15 Kohlenstoffatome und mindestens zwei Hydroxylgruppen. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispiels-weise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin.

Als Konservierungsmittel eignen sich beispielsweise Phenoxyethanol, Formaldehydlösung, Para-bene, Pentandiol oder Sorbinsäure sowie die in Anlage 6, Teil A und B der Kosmetikverordnung aufge-führten weiteren Stoffklassen. Als Insekten-Repellentien kommen N,N-Diethyl-m-touluamid, 1,2-Pentandiol oder Insect repellent 3535 in Frage, als Selbstbräuner eignet sich Dihydroxyaceton.

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxy-ethylisobutyrat, p-tert.-Butylcyclohexylacetat, Linalylacetat, Dimethylbenzylcarbinylacetat, Phenylethylacetat, Linalylbenzoat, Benzylformiat, Ethylmethyl-phenylglycinat, Allylcyclohexylpropionat, Styrallylpro-pionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, ∝-Iso-methylionon und Methylcedrylketon, zu den Alkoholen Anethol, Citronellol, Eugenol, Isoeugenol, Gera-niol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwen-det, die gemeinsam eine ansprechende Duftnote erzeugen. Auch ätherische Öle geringerer Flüch-tigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiver-öl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damas-cone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, Verlag Chemie, Weinheim, 1984, S.81-106 zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt- oder Heißprozesse erfolgen; vorzugsweise arbeitet man nach der Phaseninversionstemperatur-Methode.

Ein weiterer Gegenstand der Erfindung sind pharmazeutische Zubereitungen zur Behandlung und/oder Prophylaxe von androgen-abhängigen Störungen des Haarfollikels, insbesondere von androgenetisch bedingtem Haarausfall, enthaltend mindestens einen der erfindungsgemäßen Steroidsulfatase-Inhibitoren.

Weiterhin bevorzugt ist es, in den erfindungsgemäßen Zubereitungen zusätzlich 5-α-Reduktase Inhibitoren wie beispielsweise Finasterid einzusetzen. Vorteilhafterweise werden so die Stoffwechselwege, die zur Bildung von DHT und damit auch zur Entstehung von androgenetisch bedingtem Haarausfall führen, zu hemmen. Die pharmazeutischen Zubereitungen können insbesondere topisch auf die gefährdeten und/oder betroffenen Hautstellen, insbesondere auf die Kopfhaut und/oder die Haare selbst, aufgebracht werden.

Die pharmazeutischen Zubereitungen können Die pharmazeutischen Zubereitungen enthalten bevorzugt einen physiologisch verträglichen Träger. Dieser umfaßt ein oder mehrere Adjuvantien, wie sie üblicherweise in solchen Zubereitungen verwendet werden, wie z. B. Verdickungsmittel, anfeuchtende und/oder feuchthaltende Substanzen, Tenside, Emulgatoren, Weichmacher, Schaumbremsen, Fette, Öle, Wachse, Silikone, Sequestrierungsmittel, anionische, kationische, nichtionische oder amphotere Polymere, Alkalinisierungs- oder Acidifizierungsmittel, Alkohole, Polyole, Enthärter, Adsorbentien, Lichtschutzmittel, Elektrolyte, organische Lösungsmittel, Konservierungsmittel, Bakterizide, Antioxidantien, Duftstoffe, Aromen, Farbstoffe und Pigmente.

Bei der bevorzugten topischen Verabreichung können die pharmazeutischen Zubereitungen in unterschiedlichen Formen, wie beispielsweise Cremes oder Salben, aber auch medizinischen Shampoos oder Haarwässern vorliegen, auch in wasserfreier Form wie beispielsweise einem Öl oder einem Balsam oder auch in Form einer Öl-in-Wasser- oder Wasser-in-Öl-Emulsion, die beispielsweise eine Creme oder eine Milch sein kann, in Form von Suspensionen, Lösungen, Pudern oder Pflastern. Wenn die Zubereitungen in wasserfreier Form vorliegen, kann der Träger ein pflanzliches oder tierisches Öl, ein Mineralöl oder auch ein synthetisches Öl oder Mischungen von solchen Ölen sein.

Ein weiterer Gegenstand der vorliegenden Erfindung ist ein Verfahren zur Verminderung von Haarausfall, insbesondere androgenetisch bedingtem Haarausfall, mittels Inhibierung von Steroidsulfatase, das dadurch gekennzeichnet ist, dass eine kosmetische oder pharmazeutische Zusammensetzung, enthaltend mindestens eine Steroidsulfatase-inhibierende Substanz, ausgewählt aus funktionellen C₂-C₁₂-Carbonsäuren und deren physiologisch verträglichen Metallsalzen, Derivaten von C₃-C₉-Polyolen, Phenolderivaten, Pflanzenextrakten, Flavonoiden, 6,7-disubstituierten 2,2-Dialkylchromane oder -chromenen, Aluminiumchlorohydrat, 2-Phenylethanol, Etidronsäure, 7-Acetyl-1,1,3,4,4,6-hexamethyltetralin, Tropolonderivaten, Estern der Schwefelsäure mit alkoxylierten C₈-C₁₈-Fettalkoholen und deren physiologisch verträglichen Metallsalzen, Estern der Phosphorsäure und der Triphosphorsäure mit ein- bis sechswertigen Hydroxyverbindungen, Kieselsäureestern, aus marinen Organismen isolierbaren mycosporinähnlichen Aminosäuren (MAA) sowie quaternären Siliconverbindungen, auf die Haut, insbesondere auf die Kopfhaut, aufgetragen wird.

Die folgenden Beispiele sollen die Erfindung verdeutlichen, ohne sie hierauf zu beschränken.

### Beispielsrezepturen:

### (Alle Angaben in Gew.-%)

### 14. Sprühbare Haarkur, leave-on

| | 1.1 | 1.2 | 1.3 | 1.4 | 1.5 | 1.6 | 1.7 | 1.8 |
|---|---|---|---|---|---|---|---|---|
| MONOMULS® 60-35 C | 1,24 | 1,24 | 1,24 | 1,24 | 1,24 | 1,24 | 1,24 | 1,24 |
| EUMULGIN® B 1 | 2,76 | 2,76 | 2,76 | 2,76 | 2,76 | 2,76 | 2,76 | 2,76 |
| CETIOL® S | 9,0 | 9,0 | 9,0 | 9,0 | 9,0 | 9,0 | 9,0 | 9,0 |
| CETIOL® OE | 9,0 | 9,0 | 9,0 | 9,0 | 9,0 | 9,0 | 9,0 | 9,0 |
| DOW CORNING DC 345® | 2,0 | 2,0 | 2,0 | 2,0 | 2,0 | 2,0 | 2,0 | 2,0 |
| 2,5-DIHYDRO-5-METHOXY-2-FURANON | 2,0 | 2,0 | 2,0 | 2,0 | 2,0 | 2,0 | 2,0 | 2,0 |
| GLUADIN® WQ | 2,85 | 2,85 | 2,85 | 2,85 | 2,85 | 2,85 | 2,85 | 2,85 |
| PLANTACARE® 2000 UP | 1,00 | 1,00 | 1,00 | 1,00 | 1,00 | 1,00 | 1,00 | 1,00 |
| EMBLICA™ | 1,0 | - | - | - | - | - | - | - |
| OLEANOLINE™ DPG | - | 1,0 | - | - | - | - | - | - |
| ASCORBINSÄURE | - | - | 0,2 | - | - | - | - | - |
| POLYPLANT® ME | - | - | - | 0,7 | - | - | - | - |
| GRÜNER TEE EXTRAKT | - | - | - | - | 0,015 | - | - | - |
| YUZU-ÖL | - | - | - | - | - | 0,05 | - | - |
| KAFFEESÄURE | | | | | | | 0,02 | |
| PHENYLETHYLALKOHOL | - | - | - | - | - | - | - | 1,0 |
| WASSER DEST. | AD 100 | AD 100 | AD 100 | AD 100 | AD 100 | AD 100 | AD 100 | AD 100 |
| Viskosität mPas < 100 | | | | | | | | |

### 14.Leave-on Haarkur

| | 2.1 | 2.2 | 2.3 | 2.4 | 2.5 | 2.6 | 2.7 | 2.8 | 2.9 |
|---|---|---|---|---|---|---|---|---|---|
| DEHYQUART® F 75 | 0,7 | 0,7 | 0,7 | 0,7 | 0,7 | 0,7 | 0,7 | 0,7 | 0,7 |
| DEHYMULS® PGPH | 1,0 | 1,0 | 1,0 | 1,0 | 1,0 | 1,0 | 1,0 | 1,0 | 1,0 |
| LANETTE® O | 3,0 | 3,0 | 3,0 | 3,0 | 3,0 | 3,0 | 3,0 | 3,0 | 3,0 |
| EUTANOL® G | 0,2 | 0,2 | 0,2 | 0,2 | 0,2 | 0,2 | 0,2 | 0,2 | 0,2 |
| CETIOL® J 600 | 0,1 | 0,1 | 0,1 | 0,1 | 0,1 | 0,1 | 0,1 | 0,1 | 0,1 |
| PLANTACARE® 1200 UP | 2,5 | 2,5 | 2,5 | 2,5 | 2,5 | 2,5 | 2,5 | 2,5 | 2,5 |
| 4-HYDROXY-2,5-DIMETHYL-3(2H)-FURANON | 1,0 | 1,0 | 1,0 | 1,0 | 1,0 | 1,0 | 1,0 | 1,0 | 1,0 |
| GLUADIN® W 40 | 2,0 | 2,0 | 2,0 | 2,0 | 2,0 | 2,0 | 2,0 | 2,0 | 2,0 |
| PANTHENOL (50%) | 0,7 | 0,7 | 0,7 | 0,7 | 0,7 | 0,7 | 0,7 | 0,7 | 0,7 |
| ALPHA-HYDROXYLAURINSÄURE | 0,1 | - | - | - | - | - | - | - | - |
| LÄVULINSÄURE | - | 0,5 | - | - | - | - | - | - | - |
| ZINKLACTAT | - | - | 0,1 | - | - | - | - | - | - |
| ZINKGLUCONAT | - | - | - | 0,1 | - | - | - | - | - |
| ZINKPYRROLIDON-CARBONSÄURE | - | - | - | - | 0,05 | - | - | - | - |
| ZINKCOCOYLETHERSULFAT | - | - | - | - | - | 0,2 | - | - | - |
| ASCORBYLGLUCOSID | - | - | - | - | - | - | 1,0 | - | - |
| 2-ETHYLHEXYLGLYCERINETHER | - | - | - | - | - | - | - | 1,5 | - |
| CITRUS LIQUID EXTRACT | - | - | - | - | - | - | - | - | 0,2 |
| WASSER DEST. | AD 100 | AD 100 | AD 100 | AD 100 | AD 100 | AD 100 | AD 100 | AD 100 | AD 100 |
| PH - Wert 4 | | | | | | | | | |
| Viskosität (mPas) / Brookfield, RVF 23° C, Spindel 5, 10 Upm 6800 | | | | | | | | | |

### 14.Leave-on Haarkur

| | 3.1 | 3.2 | 3.3 | 3.4 | 3.5 | 3.6 | 3.7 |
|---|---|---|---|---|---|---|---|
| SEPIGEL® 305 | 3,0 | 3,0 | 3,0 | 3,0 | 3,0 | 3,0 | 3,0 |
| COMPERLAN® KD | 2,0 | 2,0 | 2,0 | 2,0 | 2,0 | 2,0 | 2,0 |
| DIHYDRO-3-HYDROXY-4,4-DIMETHYL-2(3H)-FURANON | 3,0 | 3,0 | 3,0 | 3,0 | 3,0 | 3,0 | 3,0 |
| PLANTACARE® 1200 UP | 0,5 | 0,5 | 0,5 | 0,5 | 0,5 | 0,5 | 0,5 |
| CETIOL® J 600 | 0,5 | 0,5 | 0,5 | 0,5 | 0,5 | 0,5 | 0,5 |
| COPHEROL® 1250 | 0,2 | 0,2 | 0,2 | 0,2 | 0,2 | 0,2 | 0,2 |
| GLUADIN® ALMOND | 3,0 | 3,0 | 3,0 | 3,0 | 3,0 | 3,0 | 3,0 |
| GLUADIN® WQ | 0,8 | 0,8 | 0,8 | 0,8 | 0,8 | 0,8 | 0,8 |
| ETHANOL | 10,0 | 10,0 | 10,0 | 10,0 | 10,0 | 10,0 | 10,0 |
| EUCAROL® AGE-EC | 1,0 | - | - | - | - | - | - |
| EUCAROL® AGE-ET | - | 0,2 | - | - | - | - | - |
| Aus MARINEN ORGANISMEN ISOLIERBARE MYCOSPORINÄHNLICHE AMINOSÄUREN (MAA) | - | - | 0,05 | - | - | - | - |
| ETIDRONSÄURE | - | - | - | 0,1 | - | - | - |
| ABIL® QUAT 3474 | - | - | - | - | 1,3 | - | - |
| FLAVONOID COMPLEX SC | - | - | - | - | - | 0,5 | - |
| ARP 100 | - | - | - | - | - | - | 1,0 |
| WASSER | AD 100 | AD 100 | AD 100 | AD 100 | AD 100 | AD 100 | AD 100 |
| PH - Wert 7 | | | | | | | |
| Viskosität (mPas)/ Brookfield RVF, 23°C, Spindel 4, 10 6700 Upm | | | | | | | |

### 14. Haarspülung

| | 4.1 | 4.2 | 4.3 | 4.4 | 4.5 | 4.6 | 4.7 | 4.8 | 4.9 |
|---|---|---|---|---|---|---|---|---|---|
| DEHYQUART® C 4046 | 4,0 | 4,0 | 4,0 | 4,0 | 4,0 | 4,0 | 4,0 | 4,0 | 4,0 |
| CETIOL® SN | 1,0 | 1,0 | 1,0 | 1,0 | 1,0 | 1,0 | 1,0 | 1,0 | 1,0 |
| GLUADIN® ALMOND | 2,1 | 2,1 | 2,1 | 2,1 | 2,1 | 2,1 | 2,1 | 2,1 | 2,1 |
| DIHYDRO-3-HYDROXY-4,4-DIMETHYL-2(3H)-FURANON | 1,5 | 1,5 | 1,5 | 1,5 | 1,5 | 1,5 | 1,5 | 1,5 | 1,5 |
| PROTECTATE HR | 0,1 | - | - | - | - | - | - | - | - |
| EDERLINE AAPP1-L | - | 0,5 | - | - | - | - | - | - | - |
| EMBLICA | - | - | 0,1 | - | - | - | - | - | - |
| EUROL BT | - | - | - | 0,1 | - | - | - | - | - |
| LIPOCHROMAN-6 | - | - | - | - | 0,05 | - | - | - | - |
| HERBALIA® OLIVE, 12 GEW.-%, WÄSSRIGE LSG. | - | - | - | - | - | 0,2 | - | - | - |
| PANTROFINA PC | - | - | - | - | - | - | 1,0 | - | - |
| ROSMARINEXTRAKT 8%NC | - | - | - | - | - | - | - | 1,5 | - |
| SEPIVINOL R | - | - | - | - | - | - | - | - | 0,2 |
| WASSER DEST. | AD 100 | AD 100 | AD 100 | AD 100 | AD 100 | AD 100 | AD 100 | AD 100 | AD 100 |
| pH - Wert 3,5 | | | | | | | | | |
| Viskosität (mPas)/ Brookfield RVF, 23°C, Spindel 4, 10 Upm 4000 | | | | | | | | | |

### 14. Haarkur

| | 5.1 | 5.2 | 5.3 | 5.4 | 5.5 | 5.6 | 5.7 | 5.8 |
|---|---|---|---|---|---|---|---|---|
| DEHYQUART® L 80 | 0,9 | 0,9 | 0,9 | 0,9 | 0,9 | 0,9 | 0,9 | 0,9 |
| LANETTE® O | 3,5 | 3,5 | 3,5 | 3,5 | 3,5 | 3,5 | 3,5 | 3,5 |
| MONOMULS® 60-35 C | 1,0 | 1,0 | 1,0 | 1,0 | 1,0 | 1,0 | 1,0 | 1,0 |
| EUMULGIN® B 2 | 0,8 | 0,8 | 0,8 | 0,8 | 0,8 | 0,8 | 0,8 | 0,8 |
| COSMEDIA® GUAR C 261 | 0,3 | 0,3 | 0,3 | 0,3 | 0,3 | 0,3 | 0,3 | 0,3 |
| TETRAHYDRO-5-OXO-2-FURAN-CARBONSÄURE, NA-SALZ | 2,0 | 2,0 | 2,0 | 2,0 | 2,0 | 2,0 | 2,0 | 2,0 |
| EMBLICA™ | 1,0 | - | - | - | - | - | - | - |
| OLEANOLINE™ DPG | - | 1,0 | - | - | - | - | - | - |
| ASCORBINSÄURE | - | - | 0,2 | - | - | - | - | - |
| POLYPLANT® ME | - | - | - | 0,7 | - | - | - | - |
| GRÜNER TEE EXTRAKT | - | - | - | - | 0,015 | - | - | - |
| YUZU-ÖL | - | - | - | - | - | 0,05 | - | - |
| KAFFEESÄURE | | | | | | | 0,02 | |
| PHENYLETHYL-ALKOHOL | - | - | - | - | - | - | - | 1,0 |
| WASSER DEST. | ad 100 | Ad 100 | ad 100 | ad 100 | ad 100 | ad 100 | ad 100 | Ad 100 |
| pH ― Wert 3,5 | | | | | | | | |

### 14. Haarmaske

| | 6.1 | 6.2 | 6.3 | 6.4 | 6.5 |
|---|---|---|---|---|---|
| DEHYQUART® F 75 | 3,0 | 3,0 | 3,0 | 3,0 | 3,0 |
| LANETTE® O | 4,0 | 4,0 | 4,0 | 4,0 | 4,0 |
| CUTINA® GMS ( | 1,0 | 1,0 | 1,0 | 1,0 | 1,0 |
| EUMULGIN® B 2 | 1,5 | 1,5 | 1,5 | 1,5 | 1,5 |
| 4-HYDROXY-2,5-DIMETHYL3(2H)-FURANON | 2,0 | 2,0 | 2,0 | 2,0 | 2,0 |
| NUTRILAN® KERATIN W | 5,0 | 5,0 | 5,0 | 5,0 | 5,0 |
| PANTHENOL | 0.8 | 0.8 | 0.8 | 0.8 | 0.8 |
| ALOE VERA GEL | 2,0 | 2,0 | 2,0 | 2,0 | 2,0 |
| Ederline AAPP1-L | 0,01 | - | - | - | - |
| Eurol BT | - | 2,0 | - | - | - |
| Sepivinol R | - | - | 0,4 | - | - |
| Rosmarinextrakt 8% NC | | | | 0,7 | - |
| Lipochroman-6 | - | - | - | - | 0,015 |
| Wasser | ad 100 | ad 100 | ad 100 | ad 100 | ad 100 |
| pH ― Wert 3 - 4 | | | | | |

### 14. Intensivhaarkur

| | 7.1 | 7.2 | 7.3 | 7.4 | 7.5 | 7.6 | 7.7 |
|---|---|---|---|---|---|---|---|
| DEHYQUART® L 80 | 2,5 | 2,5 | 2,5 | 2,5 | 2,5 | 2,5 | 2,5 |
| CUTINA® GMS | 0,5 | 0,5 | 0,5 | 0,5 | 0,5 | 0,5 | 0,5 |
| LANETTE® O | 4,0 | 4,0 | 4,0 | 4,0 | 4,0 | 4,0 | 4,0 |
| HYDAGEN® HSP | 0,5 | 0,5 | 0,5 | 0,5 | 0,5 | 0,5 | 0,5 |
| DIHYDRO-3-HYDROXY-4,4-DIMETHYL-2(3H)-FURANON | 3,0 | 3,0 | 3,0 | 3,0 | 3,0 | 3,0 | 3,0 |
| LAMESOFT® PO 65 | 2,5 | 2,5 | 2,5 | 2,5 | 2,5 | 2,5 | 2,5 |
| EUCAROL® AGE-EC | 1,0 | - | - | - | - | - | - |
| EUCAROL® AGE-ET | - | 0,2 | - | - | - | - | - |
| A US MARINEN ORGANISMEN ISOLIERBARE MYCOSPORINÄHNLICHE AMINOSÄUREN (MAA) | - | - | 0,05 | - | - | - | - |
| ETIDRONSÄURE | - | - | - | 0,1 | - | - | - |
| ABIL® QUAT 3474 | - | - | - | - | 1,3 | - | - |
| FLAVONOID COMPLEX SC | - | - | - | - | - | 0,5 | - |
| ARP 100 | - | - | - | - | - | - | 1,0 |
| WASSER | AD 100 | AD 100 | AD 100 | AD 100 | AD 100 | AD 100 | AD 100 |
| PH - Wert 3,5 | | | | | | | |
| Viskosität (mPas), Brook.RVF, 23° C, Spindel 4, 10 4400 | | | | | | | |
| Upm | | | | | | | |

### 14. Haarfluid

| | 8.1 | 8.2 | 8.3 | 8.4 | 8.5 | 8.6 | 8.7 | 8.8 |
|---|---|---|---|---|---|---|---|---|
| HYDAGEN® HCMF | 0,4 | 0,4 | 0,4 | 0,4 | 0,4 | 0,4 | 0,4 | 0,4 |
| GLYKOLSÄURE | 0,2 | 0,2 | 0,2 | 0,2 | 0,2 | 0,2 | 0,2 | 0,2 |
| GLYCERIN 86% | 55,0 | 55,0 | 55,0 | 55,0 | 55,0 | 55,0 | 55,0 | 55,0 |
| TYLOSE® H 100.000 YP | 0,4 | 0,4 | 0,4 | 0,4 | 0,4 | 0,4 | 0,4 | 0,4 |
| R-(-)-2-HYDROXY-3,3-DIMETHYL-γ-BUTYROLACTON | 3,0 | 3,0 | 3,0 | 3,0 | 3,0 | 3,0 | 3,0 | 3,0 |
| GLUADIN® R | 4,0 | 4,0 | 4,0 | 4,0 | 4,0 | 4,0 | 4,0 | 4,0 |
| PANTHENOL 50% | 1,0 | 1,0 | 1,0 | 1,0 | 1,0 | 1,0 | 1,0 | 1,0 |
| ETHANOL | 10,0 | 10,0 | 10,0 | 10,0 | 10,0 | 10,0 | 10,0 | 10,0 |
| ALUMINIUMCHLORO HYDRAT | - | - | 1,0 | - | - | - | - | - |
| WILLOWHERB EXTRACT | 1,0 | - | - | - | - | - | - | - |
| MEERESALGENEXTR AKT | - | 0,6 | - | - | - | - | - | - |
| SEPIVINOL R | - | - | - | 1,0 | - | - | - | - |
| LIPOCHROMAN-6 | - | - | - | - | 1,2 | - | - | - |
| PANTROFINA PC | - | - | - | - | - | 1,7 | - | - |
| ROSMARINSÄURE | - | - | - | - | - | - | 0,01 | - |
| EDERLINE AAPP1-H | - | - | - | - | - | - | - | 1,0 |
| WASSER | AD 100 | AD 100 | AD 100 | AD 100 | AD 100 | AD 100 | AD 100 | AD 100 |
| PH ― Wert 4,5 | | | | | | | | |

### 14. Leave-on Haarmilch

| | 9.1 | 9.2 | 9.3 | 9.4 | 9.5 | 9.6 | 9.7 | 9.8 | 9.9 |
|---|---|---|---|---|---|---|---|---|---|
| DEHYQUART® L 80 | 2,0 | 2,0 | 2,0 | 2,0 | 2,0 | 2,0 | 2,0 | 2,0 | 2,0 |
| LAMESOFT® PO 65 | 2,0 | 2,0 | 2,0 | 2,0 | 2,0 | 2,0 | 2,0 | 2,0 | 2,0 |
| TETRAHYDRO-5-OXO-2-FURANCARBONSÄURE,K-SALZ | 2,0 | 2,0 | 2,0 | 2,0 | 2,0 | 2,0 | 2,0 | 2,0 | 2,0 |
| FIXOLIDE® NP | 0,01 | - | - | - | - | - | - | - | - |
| NATRIUMLAUROYLGLUTAMAT | - | 2,0 | - | - | - | - | - | - | - |
| SALICYLSÄURE | - | - | 0,4 | - | - | - | - | - | - |
| CRODAFOS® SG | | | | 0,7 | - | - | - | - | - |
| MATE-TEE-EXTRAKT (DRAGOCO) | - | - | - | - | 0,01 5 | - | - | - | - |
| TINOGARD™ TT DD | - | - | - | - | - | 0,1 | - | - | 0,1 |
| PHENOXYETHANOL | - | - | - | - | - | - | 0,5 | - | 0,5 |
| PHENOXYETHANOLKIESEL-SÄUREESTER | - | - | - | - | - | - | - | 0,05 | 0,05 |
| WASSER | AD 100 | AD 100 | AD 100 | AD 100 | AD 100 | AD 100 | AD 100 | AD 100 | AD 100 |

### 14. Pumpspray-Festiger

| | 10.1 | 10.2 | 10.3 | 10.4 | 10.5 | 10.6 |
|---|---|---|---|---|---|---|
| HYDAGEN® HCMF | 1,0 | 1,0 | 1,0 | 1,0 | 1,0 | 1,0 |
| GLYKOLSÄURE | 0,4 | 0,4 | 0,4 | 0,4 | 0,4 | 0,4 |
| 2,5-DIHYDRO-5-METHOXY-2-FURANON | 0,5 | 0,5 | 0,5 | 0,5 | 0,5 | 0,5 |
| PLANTACARE® 1200 U P | 0,2 | 0,2 | 0,2 | 0,2 | 0,2 | 0,2 |
| GLUADIN® WQ | 1,0 | 1,0 | 1,0 | 1,0 | 1,0 | 1,0 |
| ETHANOL | 40,0 | 40,0 | 40,0 | 40,0 | 40,0 | 40,0 |
| EUROL BT | 1,0 | - | - | - | - | - |
| PANTROFINA PC | - | 0,2 | - | - | - | - |
| LIPOCHROMAN-6 | - | - | 0,05 | - | - | - |
| SEPIVINOL R | - | - | - | 0,1 | - | - |
| EDERLINE AAPP1-H | - | - | - | - | 0,5 | - |
| EDERLINE AAPP1-L | - | - | - | - | - | 1,0 |
| WASSER | AD 100 | AD 100 | AD 100 | AD 100 | AD 100 | AD 100 |
| pH Wert 4,0 | | | | | | |

### 14. Schaumfestiger

| | 11.1 | 11.2 | 11.3 | 11.4 | 11.5 | 11.6 | 11.7 |
|---|---|---|---|---|---|---|---|
| HYDAGEN® HCMF | 0,4 | 0,4 | 0,4 | 0,4 | 0,4 | 0,4 | 0,4 |
| GLYKOLSÄURE | 0,2 | 0,2 | 0,2 | 0,2 | 0,2 | 0,2 | 0,2 |
| DEHYQUART® A | 1,0 | 1,0 | 1,0 | 1,0 | 1,0 | 1,0 | 1,0 |
| GLUADIN® W 40 | 2,0 | 2,0 | 2,0 | 2,0 | 2,0 | 2,0 | 2,0 |
| EDERLINE AAPP1-H | 1,0 | - | - | - | - | - | - |
| HERBALIA® OLIVE, 12 GEW.-%, WÄSSRIGE LSG. | - | 0,2 | - | - | - | - | - |
| LIPOCHROMAN-6 | - | - | 0,7 | - | - | - | - |
| EDERLINE AAPP1-L | - | - | - | 0,015 | - | - | - |
| SEPIVINOL R | - | - | - | - | 0,05 | - | - |
| PANTROFINA PC | | | | | | 0,02 | |
| EUROL BT | - | - | - | - | - | - | 1,0 |
| WASSER DEST. | AD 100 | AD 100 | AD 100 | AD 100 | AD 100 | AD 100 | AD 100 |

### 14.2-in-1 Shampoo

| | 12.1 | 12.2 | 12.3 | 12.4 | 12.5 | 12.6 | 12.7 | 12.8 | 12.9 |
|---|---|---|---|---|---|---|---|---|---|
| TEXAPON® N 70 | 12, 0 | 12, 0 | 12, 0 | 12, 0 | 12, 0 | 12, 0 | 12, 0 | 12, 0 | 12, 0 |
| DEHYTON® PK 45 | 2,5 | 2,5 | 2,5 | 2,5 | 2,5 | 2,5 | 2,5 | 2,5 | 2,5 |
| PLANTACARE® 818 UP | 3,0 | 3,0 | 3,0 | 3,0 | 3,0 | 3,0 | 3,0 | 3,0 | 3,0 |
| LAMESOFT® PO 65 | 3,0 | 3,0 | 3,0 | 3,0 | 3,0 | 3,0 | 3,0 | 3,0 | 3,0 |
| COSMEDIA® GUAR C 261 N | 0,3 | 0,3 | 0,3 | 0,3 | 0,3 | 0,3 | 0,3 | 0,3 | 0,3 |
| EUPERLAN® PK 1200 | 5,0 | 5,0 | 5,0 | 5,0 | 5,0 | 5,0 | 5,0 | 5,0 | 5,0 |
| NATRIUMCHLORID | 1,2 | 1,2 | 1,2 | 1,2 | 1,2 | 1,2 | 1,2 | 1,2 | 1,2 |
| D,L-4-HYDROXYMETHYL-γ-BUTYROLACTON | 0,3 | 0,3 | 0,3 | 0,3 | 0,3 | 0,3 | 0,3 | 0,3 | 0,3 |
| EUXYL® K 400 | 0,1 | 0,1 | 0,1 | 0,1 | 0,1 | 0,1 | 0,1 | 0,1 | 0,1 |
| ALPHA-HYDROXY-LAURINSÄURE | 0,1 | - | - | - | - | - | - | - | - |
| LÄVULINSÄURE | - | 0,5 | - | - | - | - | - | - | - |
| ZINKLACTAT | - | - | 0,1 | - | - | - | - | - | - |
| ZINKGLUCONAT | - | - | - | 0,1 | - | - | - | - | - |
| ZINKPYRROLIDON-CARBONSÄURE | - | - | - | - | 0,05 | - | - | - | - |
| ZINKCOCOYLETHER-SULFAT | - | - | - | - | - | 0,2 | - | - | - |
| ASCORBYLGLUCOSID | - | - | - | - | - | - | 1,0 | - | - |
| 2-ETHYLHEXYL-GLYCERINETHER | - | - | - | - | - | - | - | 1,5 | - |
| CITRUS LIQUID EXTRACT | - | - | - | - | - | - | - | - | 0,2 |
| WASSER DEST. | AD 100 | AD 100 | AD 100 | AD 100 | AD 100 | AD 100 | AD 100 | AD 100 | AD 100 |
| PH ― Wert 5,5 | | | | | | | | | |
| Viskosität (mPas), Brookfield RFT, 23°C, Sp.4, 10 Upm 6300 | | | | | | | | | |

### 14. Konditioniershampoo

| | 13.1 | 13.2 | 13.3 | 13.4 | 13.5 | 13.6 | 13.7 | 13.8 |
|---|---|---|---|---|---|---|---|---|
| TEXAPON® N 70 | 10,0 | 10,0 | 10,0 | 10,0 | 10,0 | 10,0 | 10,0 | 10,0 |
| PLANTACARE® 818 UP | 4,0 | 4,0 | 4,0 | 4,0 | 4,0 | 4,0 | 4,0 | 4,0 |
| DEHYTON® K | 5,0 | 5,0 | 5,0 | 5,0 | 5,0 | 5,0 | 5,0 | 5,0 |
| LAMESOFT® PO 65 | 1,5 | 1,5 | 1,5 | 1,5 | 1,5 | 1,5 | 1,5 | 1,5 |
| EUPERLAN® PK 3000 AM | 3,2 | 3,2 | 3,2 | 3,2 | 3,2 | 3,2 | 3,2 | 3,2 |
| DIHYDRO-3-HYDROXY-4,4-DIMETHYL-2(3H)-FURANON | 1,5 | 1,5 | 1,5 | 1,5 | 1,5 | 1,5 | 1,5 | 1,5 |
| POLYMER JR® 400 | 0,3 | 0,3 | 0,3 | 0,3 | 0,3 | 0,3 | 0,3 | 0,3 |
| NATRIUMCHLORID | 1,5 | 1,5 | 1,5 | 1,5 | 1,5 | 1,5 | 1,5 | 1,5 |
| EMBLICA™ | 1,0 | - | - | - | - | - | - | - |
| OLEANOLINE™ DPG | - | 1,0 | - | - | - | - | - | - |
| ASCORBINSÄURE | - | - | 0,2 | - | - | - | - | - |
| POLYPLANT® ME | - | - | - | 0,7 | - | - | - | - |
| GRÜNER TEE EXTRAKT | - | - | - | - | 0,015 | - | - | - |
| YUZU-ÖL | - | - | - | - | - | 0,05 | - | - |
| KAFFEESÄURE | | | | | | | 0,02 | |
| PHENYLETHYLALKOHOL | - | - | - | - | - | - | - | 1,0 |
| WASSER DEST. | AD 100 | AD 100 | AD 100 | AD 100 | AD 100 | AD 100 | AD 100 | AD 100 |
| PH - Wert 5,5 | | | | | | | | |
| Viskosität (mPas), Brookfield RVF, 23°C, Spindel 4, 10 8500 | | | | | | | | |
| Upm | | | | | | | | |

### 14. sehr mildes Shampoo

| | 14.1 | 14.2 | 14.3 | 14.4 | 14.5 | 14.6 | 14.7 | 14.8 | 14.9 |
|---|---|---|---|---|---|---|---|---|---|
| POLYMER® JR 400 | 0,4 | 0,4 | 0,4 | 0,4 | 0,4 | 0,4 | 0,4 | 0,4 | 0,4 |
| TEXAPON® K 14 S SPECIAL 70% | 11,0 | 11,0 | 11,0 | 11,0 | 11,0 | 11,0 | 11,0 | 11,0 | 11,0 |
| DEHYTON® PK 45 | 5,0 | 5,0 | 5,0 | 5,0 | 5,0 | 5,0 | 5,0 | 5,0 | 5,0 |
| PLANTACARE® 818 UP | 5,0 | 5,0 | 5,0 | 5,0 | 5,0 | 5,0 | 5,0 | 5,0 | 5,0 |
| LAMESOFT® PO 65 | 5,0 | 5,0 | 5,0 | 5,0 | 5,0 | 5,0 | 5,0 | 5,0 | 5,0 |
| EUXYL® K 400 | 0,1 | 0,1 | 0,1 | 0,1 | 0,1 | 0,1 | 0,1 | 0,1 | 0,1 |
| NATRIUMCHLORID | 1,8 | 1,8 | 1,8 | 1,8 | 1,8 | 1,8 | 1,8 | 1,8 | 1,8 |
| FIXOLIDE® NP | 0,01 | - | - | - | - | - | - | - | - |
| NATRIUMLAUROYLGLUTA MAT | - | 2,0 | - | - | - | - | - | - | - |
| SALICYLSÄURE | - | - | 0,4 | - | - | - | - | - | - |
| CRODAFOS® SG | | | | 0,7 | - | - | - | - | - |
| MATE-TEE-EXTRAKT | - | - | - | - | 0,01 5 | - | - | - | - |
| TINOGARD™ TT DD | - | - | - | - | - | 0,1 | - | - | 0,1 |
| PHENOXYETHANOL | - | - | - | - | - | - | 0,5 | - | 0,5 |
| PHENOXYETHANOLKIESE LSÄUREESTER | - | - | - | - | - | - | - | 0,05 | 0,05 |
| WASSER | AD 100 | AD 100 | AD 100 | AD 100 | AD 100 | AD 100 | AD 100 | AD 100 | AD 100 |
| PH - Wert 5,5 | | | | | | | | | |
| Viskosität (mPas), Brookfield RVF, 23°C, Spindel 4, 10 3900 | | | | | | | | | |
| Upm | | | | | | | | | |

### 14. Perlglänzendes Pflegeshampoo

| | 15.1 | 15.2 | 15.3 | 15.4 | 15.5 | 15.5 |
|---|---|---|---|---|---|---|
| TEXAPON® NSO | 29,0 | 29,0 | 29,0 | 29,0 | 29,0 | 29,0 |
| PLANTACARE® 818 UP | 5,0 | 5,0 | 5,0 | 5,0 | 5,0 | 5,0 |
| TEXAPON® SB 3 KC | 3,8 | 3,8 | 3,8 | 3,8 | 3,8 | 3,8 |
| HYDAGEN® HSP | 0,5 | 0,5 | 0,5 | 0,5 | 0,5 | 0,5 |
| EUPERLAN® PK 3000 AM | 3,0 | 3,0 | 3,0 | 3,0 | 3,0 | 3,0 |
| NATRIUMCHLORID | 2,0 | 2,0 | 2,0 | 2,0 | 2,0 | 2,0 |
| ALUMINIUMCHLOROHYDRAT | 0,6 | | | | | |
| EDERLINE AAPP1-L | | 0,01 | - | - | - | - |
| EUROL BT | - | - | 2,0 | - | - | - |
| SEPIVINOL R | - | - | - | 0,4 | - | - |
| ROSMARINEXTRAKT 8% NC | | | | | 0,7 | - |
| LIPOCHROMAN-6 | - | - | - | - | - | 0,015 |
| WASSER | AD 100 | AD 100 | AD 100 | AD 100 | AD 100 | AD 100 |
| PH - Wert 5,5 | | | | | | |
| Viskosität (mPas), Brook.RVF, 23° C, Spindel 4, 10 4100 | | | | | | |
| Upm | | | | | | |

| Liste der eingesetzten Rohstoffe | | |
|---|---|---|
| DC® 245 | Cyclopentasiloxan | Dow Corning |
| Citrus Liquid Extract | Citrusfruchtsamenextrakt in Glycerin im Verhältnis 1:1 | Centroflora, Brazil |
| Crodafos® SG | PPG-5-Ceteth-10 Phosphate | Croda Oleochemicals |
| Sensiva® SC 50 | 2-Ethylhexylglycerinether | Schülke & Mayr |
| Polyplant® ME | Melissenextrakt | Polygon |
| Grüner Tee Extrakt | Pulverförmig | Dragoco |
| Mate / Mate-Tee-Extrakt | Pulverförmig | Dragoco |
| Yuzu-Öl | | Takasago |
| Eucarol® AGE-EC | Disodium Cocopolyglucose Citrate | Cesalpinia Chemicals |
| Eucarol® AGE-ET | Sodium Alkylpolyglucose Tartrate | Cesalpinia Chemicals |
| Flavonoid Complex SC | Water, Butylene Glycol, Gingko Biloba Leaf Extract, Phenoxyethanol, Methyl Paraben, Ethyl Paraben, Propyl Paraben, Butyl Paraben, Isobutyl Paraben, ca. 2 % Flavonoid-Gehalt | Cosmetochem |
| ARP 100 | Extrakt aus den Beeren von Saw Palmetto | Greentech/Rahn |
| Abil® Quat 3474 | Quaternium-80 | Th. Goldschmidt |
| Fixolide® NP | 7-Acetyl-1,1,3,4,4,6-hexamethyltetralin | Givaudan |
| Emblica™ | Extrakt aus den Beeren von Phyllanthus emblica, | Merck KgaA |
| Oleanoline™ DPG | Extrakt aus Olivenbaumblättern in Dipropylenglycol | Impag Seporga |
| Cibafast™ H | Sodium Benzotriazolyl Butylphenol Sulfonate, Buteth-3, Tributyl Citrate | Ciba |
| Tinogard™ TT DD | Tetradibutyl Pentaerithrityl Hydroxyhydrocinnamate | Ciba |
| Eurol BT | Olive Leaf Extract, Water | B & T Srl, Milan |
| Lipochroman-6 | Dimethylmethoxy Chromanol | Merck KGaA |
| Oleanoline | Extrakt aus Olivenbaumblättern in Dipropylenglycol | Cognis |
| Herbalia® Olive | Olea Europea (Olive) Leaf Extract (and) Maltodextrin (and) Srlica | Cognis |
| Pantrofina OC | Extrakt aus Pinienrinde (Pinus Pinaster) | Res Pharma Srl, Milan |
| Protectate HR | Riechstoffgemisch | Symrise (vormals Haarmann & Reimer) |
| Sepivinol R | Rotwein-Extrakt | Seppic |
| Weidenröschen-Extrakt | Willowherb Extract | Symrise (Dragoco) |
| Rosmarinextrakt 8% NC | Rosmarinextrakt wasserlöslich Pulver mit Standardmenge an 5% Rosmarinsäure, 3% Apigenin-7-glycosid | Dr. Marcus |
| MONOMULS® 60-35 C | Hydrierte Palm Glyceride | Cognis |
| EUMULGIN® B 1 | Polyoxyethylen-12-Cetylstearylalkohol | Cognis |
| CETIOL® S | Kohlenwasserstoff | Cognis |
| CETIOL® OE | Di-n-octyl Ether | Cognis |
| Dow Corning DC 345®, | | Dow Corning |
| GLUADIN® WQ | Kationisiertes Weizenproteinhydrolysat (ca. 31%) | Cognis |
| DEHYQUART® F 75 | Mischung aus Esterquat und Fettalkohol | Cognis |
| DEHYMULS® PGPH | Polyglycerinpoly-12-hydroxystearat | Cognis |
| LANETTE® O | Cetylstearylalkohol | Cognis |
| EUTANOL® G | 2-Octyldodecanol (Guerbet-Alkohol) | Cognis |
| CETIOL® J 600 | Flüssiger Wachsester | Cognis |
| PLANTACARE® 1200 UP | C 12 - C 16 Fettalkoholglycosid (ca. 50%) | Cognis |
| Sepigel® 305 | Polyacrylamide (and) C13-14 Isoparaffin (and) Laureth-7 | Seppic |
| COMPERLAN® KD | Kokosfettsäurediethanol-amid | Cognis |
| Dihydro-3-hydroxy-4,4-dimethyl-2(3H)-furanon | | Aldrich |
| PLANTACARE® 1200 UP | C 12 - C 16 Fettalkoholglycosid (ca. 50%) | Cognis |
| COPHEROL® 1250 | RRR-(α)-Tocopherylacetat | Cognis |
| DEHYQUART® C 4046 | Cetearyl Alcohol (and) Dipalmitoylethyl Hydroxyethylmonium Methosulfate (and) Ceteareth20 | Cognis |
| Dehyquart® L 80 | Mischung aus Esterquat und Propyleneglykol (ca. 75%) Dicocoylethyl Hydroxyethylmonium Methosulfate (and) Propylene Glycol | Cognis |
| EUMULGIN® B 2 | Polyoxyethylen-20-Cetylstearylalkohol | Cognis |
| COSMEDIA® GUAR C 261 | Guarhydroxypropyl-trimethyl-ammonium Chlorid | Cognis |
| NUTRILAN® KERATIN W | Partialhydrolysat aus Keratin (ca. 20%) | Cognis |
| CUTINA® GMS | Glycerinmonostearat | Cognis |
| HYDAGEN® HSP | Trimethylolpropane-Hydroxymethylstearate-Ether | Cognis |
| HYDAGEN® HCMF | Chitosan Pulver | Cognis |
| DEHYQUART® L 80 | Dicocoylethyl Hydroxyethylmonium Methosulfate (and) Propylene Glycol (ca. 75%) | Cognis |
| GLYKOLSÄURE | | MERCK |
| TYLOSE® H 100.000 YP | | HOECHST |
| R-(-)-2-HYDROXY-3,3-DIMETHYL-γ-BUTYROLACTON | | ALDRICH |
| GLUADIN® R | Partialhydrolysat aus Reis (ca. 27%) | Cognis |
| CETIOL® SN | Ester einer verzweigten Fettsäure mit gesättigten Fettalkoholen C 16 - C 18, Cetearyl Isononanoate | Cognis |
| CUTINA® MD | Gemisch aus Mono- und Diglyceriden der Palmitin- und Stearinsäure | Cognis |
| EUMULGIN® B 1 | Polyoxyethylen-12-Cetylstearylalkohol | Cognis |
| CETIOL® V | Ölsäuredecylester | Cognis |
| D,L-4-Hydroxymethyl-γ-butyrolacton | | Merck |
| DEHYTON® PK 45 | Fettsäure-amid- Derivat mit Betain-struktur (ca. 45%) | Cognis |
| EUPERLAN® PK 1200 | Coco-Glucoside (and) Glycol Distearate (and) Glycerin | Cognis |
| EUPERLAN® PK 3000 AM | Glycol Distearate (and) Laureth 4 (and) Cocamidopropyl Betaine | Cognis |
| D,L-4-Hydroxymethyl-γ-butyrolacton | | Merck |
| Euxyl® K 400 | 1,2-Dibromcyanobutan und 2-Phenoxyethanol | Schülke & Mayr |
| TEXAPON® N 70 | Natriumlaurylethersulfat mit 2 Mol EO (ca. 70%) | Cognis |
| DEHYTON® K | Fettsäure-amid- Derivat mit Betain-struktur (ca. 30%) | Cognis |
| Dihydro-3-hydroxy-4,4-dimethyl-2(3H)-furanon | | Aldrich |
| Polymer JR® 400 | Polyquaternium 10 | Amerchol |
| TEXAPON® K 14 S Special 70% | Natriumlaurylmyristylethersulfat (ca. 70%) | Cognis |
| PLANTACARE® 818 UP | C 8 - C 16 Fettalkohol-glycosid (ca. 50%) | Cognis |
| DEHYQUART® A | Cetyltrimethylammonium-chlorid (ca. 25%) | Cognis |
| GLUADIN® W 40 | Partialhydrolysat aus Weizen (ca. 40%) | Cognis |
| TEXAPON® SB 3 KC | Sulfobernsteinsäurehalb-ester auf Basis eines Alkylpolyglycolethers, Di-Na-Salz (ca. 40%) | Cognis |

### Überprüfung der inhibitorischen Wirkung der Steroidsulfatase-Inhibitoren (in vitro)

Da die Sulfatase des Haarfollikels nicht zur Verfügung stand, diente eine handelsübliche Arylsulfatase (EC 3.1.6.1) aus *Aerobacter aerogenes* als Modellenzym, um die Hemmwirkung der erfindungsgemäß verwendeten Inhibitoren zu testen. Die Tests wurden mit dem Sulfatase-Enzymassay Product No. S-1629 von Sigma gemäß den Angaben im *Sigma Quality Control Test* Procedure-Datenblatt durchgeführt. Als Referenz-Inhibitoren dienten Ascorbinsäure und ATP.

Zur Auswertung wurde die Arylsulfatase-katalysierte Bildung von p-Nitrophenol aus dem Substrat p-Nitrophenylsulfat (pNPS) spektrophotometrisch (λ = 420 nm) verfolgt.
Die Reaktionslösungen von 1000 µl, die auf eine Reaktionstemperatur von 37°C temperiert wurden, enthielten 187 mM Tris/HCl (pH 7,1), 8 mM pNPS und eine Startkonzentration von 0,05 U/ml Arylsulfatase. Die Einheit U der Enzymaktivität wurde so definiert, dass 1 U Sulfatase 1,0 µmol pNPS pro Minute bei pH 7,1 und 37°C hydrolysiert.
Die Testdurchführung ist auch offenbart bei H.R. Fowler und D. H. Rammler, Biochemistry 3, 230, 1964.
Die Reaktion wurde durch Zugabe des Enzyms gestartet und der Anstieg der Absorption bei λ = 420 nm über 5 Minuten verfolgt. Der lineare Anstieg der Absorption (A) pro Zeiteinheit (t) ist hierbei ein Maß für die Aktivität des Enzyms (ΔA/Δt). Die Aktivität des Enzyms in Abwesenheit eines Inhibitors, (ΔA₁/Δt₁ ), wurde als Referenz gleich 100% gesetzt. Unter analogen Bedingungen wurden die Aktivitäten in Gegenwart eines Inhibitors (ΔA₂/Δt₂) bestimmt. Die Hemmwirkung des Inhibitors bzw. die Minderung der Enzymaktivität wurde dann berechnet gemäß der Formel 100% - (ΔA₂/Δt₂ )/( ΔA₁/Δt₁ )%.

**Tabelle 1:**

| Aluminiumchlorohydrat (ACH) als Inhibitor im Arylsulfatase-Enzymtest | |
|---|---|
| ACH-Lösung Locron® L (v/v*) | Hemmwirkung |
| 0% | 0% |
| 0,01 % | Keine Hemmung meßbar |
| 0,1 % | 6 % |
| 0,5 % | 27 % |
| 1 % | 52 % |

| | |
|---|---|
| *: volume per volume Locron® L: 50 % w/w wässrige Lösung von Aluminiumchlorohydrat (Clariant) | |

**Tabelle 2:**

| Verschiedene Wirkstoffe als Inhibitor im Arylsulfatase-Enzymtest | |
|---|---|
| **Rohstoff** | **Konzentration mit einer inhibierenden Wirkung von 50 %** [Gew.-% Rohstoff tel quel in wässriger Lösung] |
| Ederline AAPP1-H | 1,0 |
| Ederline AAPP1-L | 0,4 |
| Emblica | 0,025 |
| Eurol BT | 0,05 |
| Lipochroman-6 | 0,1 |
| Herbalia® Olive | 0,04 |
| Pantrofina PC | 0,4 |
| Rosmarinextrakt 8%NC | 0,005 |
| Sepivinol | 0,0025 |

## Patentansprüche

1. Verwendung von mindestens einer Steroidsulfatase-inhibierenden Substanz, ausgewählt aus funktionellen C₂-C₁₂-Carbonsäuren und deren physiologisch verträglichen Metallsalzen, Derivaten von C₃-C₉-Polyolen, Phenolderivaten, Pflanzenextrakten, Riechstoffen, Flavonoiden, Isoflavonoiden, 6,7-disubstituierten 2,2-Dialkylchromanen oder -chromenen, Aluminiumchlorohydrat, 2-Phenylethanol, Etidronsäure, 7-Acetyl-1,1,3,4,4,6-hexamethyltetralin, Tropolonderivaten, Estern der Schwefelsäure mit alkoxylierten C₈-C₁₈-Fettalkoholen und deren physiologisch verträglichen Metallsalzen, Estern der Phosphorsäure und der Triphosphorsäure mit ein- bis sechswertigen Hydroxyverbindungen, Kieselsäureestern, aus marinen Organismen isolierbaren mycosporin-ähnlichen Aminosäuren (MAA) sowie quaternären Siliconverbindungen in einer kosmetischen oder pharmazeutischen-Zusammensetzung zur Verminderung von Haarausfall.

2. Verwendung gemäß Anspruch 1, **dadurch gekennzeichnet, dass** die funktionellen C₂-C₁₂-Carbonsäuren ausgewählt sind aus C₂-C₁₂-Hydroxycarbonsäuren, C₃-C₁₂-Ketocarbonsäuren, C₂-C₅-Aminosäuren und deren N-C₂-C₁₂-Acylderivaten sowie den physiologisch verträglichen Metallsalzen dieser Säuren.

3. Verwendung gemäß einem der vorhergehenden Ansprüche, **dadurch gekennzeichnet, dass** die physiologisch verträglichen Metallsalze ausgewählt sind aus den Alkalimetall-, Magnesium-, Calcium-, Aluminium-, Zink- und Mangan-Salzen.

4. Verwendung gemäß einem der vorhergehenden Ansprüche, **dadurch gekennzeichnet, dass** die C₂-C₁₂-Hydroxycarbonsäuren und ihre physiologisch verträglichen Metallsalze ausgewählt sind aus alpha-Hydroxylaurinsäure, alpha-Hydroxycaprinsäure, alpha-Hydroxycaprylsäure, Zinklactat, Aluminiumlactat, Aluminiumgluconat, Zinkgluconat, Mangangluconat und Magnesiumglucoheptonat.

5. Verwendung gemäß Anspruch 2, **dadurch gekennzeichnet, dass** die C₃-C₁₂-Ketocarbonsäuren ausgewählt sind aus Lävulinsäure.

6. Verwendung gemäß Anspruch 2, **dadurch gekennzeichnet, dass** die C₂-C₅-Aminosäuren, deren N-C₂-C₁₂-Acylderivate sowie deren physiologisch verträgliche Metallsalze ausgewählt sind aus Aluminiumglycinat, Zinkpyroglutamat, Natriumoctanoylglutamat, Natriumdecanoylglutamat, Natriumlauroylglutamat, Natriummyristoylglutamat, Natriumcetoylglutamat und Natriumstearoylglutamat.

7. Verwendung gemäß Anspruch 1, **dadurch gekennzeichnet, dass** die Derivate von C₃-C₉-Polyolen ausgewählt sind aus Ascorbinsäure, Monoalkyl-C₃-C₉-Polyolethern, Ethern und Estern von gewünschtenfalls alkylierter Glucose sowie den Tetraestern von Pentaerythrit mit einen substituierten Arylrest enthaltenden C₂-C₄-Carbonsäuren.

8. Verwendung gemäß Anspruch 7, **dadurch gekennzeichnet, dass** die Monoalkyl-C₃-C₉-Polyolether ausgewählt sind aus Hexylglycerinether, 2-Ethylhexylglycerinether, 2-Ethyloctylglycerinether und 2-Ethyldecylglycerinether.

9. Verwendung gemäß Anspruch 7, **dadurch gekennzeichnet, dass** die Ether und Ester von gewünschtenfalls alkylierter Glucose ausgewählt sind aus Ascorbylglucosid, Dinatrium-Kokosalkylpolyglucosidcitrat (Disodium Cocopolyglucose Citrate) und Natrium-Kokosalkylpolyglucosidtartrat (Sodium Cocopolyglucose Tartrate).

10. Verwendung gemäß Anspruch 7, **dadurch gekennzeichnet, dass** die Tetraester von Pentaerythrit mit einen substituierten Arylrest enthaltenden C₂-C₄-Carbonsäuren ausgewählt sind aus Pentaerythrittetrakis[3(3,5-di-tert.-butyl-4-hydroxyphenyl)propionat].

11. Verwendung gemäß Anspruch 1, **dadurch gekennzeichnet, dass** die Phenolderivate ausgewählt sind aus Phenoxyethanol, Salicylsäure, Rosmarinsäure, Kaffeesäure, Tocopherolen, Tocopherylestern, Benzotriazolyl-substituierten 4-Phenolsulfonsäuren und deren Salzen sowie Polyphenolen.

12. Verwendung gemäß Anspruch 11, **dadurch gekennzeichnet, dass** die Polyphenole ausgewählt sind aus Brenzcatechin, Resorcin, Hydrochinon, Phloroglucin, Pyrogallol, Hexahydroxybenzol, Anthocyanidinen, Flavonen, Gerbstoffen (Catechinen, Tanninen), Usninsäure, Acylpolyphenolen, den Derivaten der Gallussäure, den Derivaten der Digallussäure und der Digalloylgallussäure sowie den Polyphenolen aus Trauben und aus Rotwein.

13. Verwendung gemäß Anspruch 1, **dadurch gekennzeichnet, dass** die Pflanzenextrakte ausgewählt sind aus den Extrakten von Melisse, Grünem Tee (Camellia sinensis), Mate (Ilex paraguayensis), Japanischem Tee (Camellia japanensis), aus den Früchten (Beeren) der Fächerpalme oder Sägepalme (Saw Palmetto, Serenoa repens), aus Gingko biloba, Yuzu-Öl, aus Citrusfruchtsamen, aus den Früchten (Beeren) von Phyllanthus emblica, aus den Blättern des Olivenbaumes (Olea europaea) sowie aus Extrakten von Meeresalgen, Apfelkernen, Pinienrinde (Pinus Pinaster), Trauben, Rotwein, Rosmarin, Hyssop (Ysop), Gewürznelke und Weidenröschen (Epilobium angustifolium, Willowherb).

14. Verwendung gemäß Anspruch 1, **dadurch gekennzeichnet, dass** die Riechstoffe ausgewählt sind aus dem Rohstoff Protectate HR.

15. Verwendung gemäß Anspruch 1, **dadurch gekennzeichnet, dass** die Flavonoide ausgewählt sind aus Apigenin-7-glucosid, α-Glucosylrutin, α-Glucosylmyricetin, α-Glucosylisoquercetin, α-Glucosylquercetin, Naringin, Hesperidin, Neohesperidin, Rutin, Troxerutin, Monoxerutin, Diosmin, Eriodictin, Phlorizin und Neohesperidindihydrochalkon.

16. Verwendung gemäß Anspruch 1, **dadurch gekennzeichnet, dass** die Isoflavonoide ausgewählt sind aus Genistein, Daidzein, Glycitein, Formononetin, Genistin und Daidzin.

17. Verwendung gemäß Anspruch 1, **dadurch gekennzeichnet, dass** die 6,7-disubstituierten 2,2-Dialkylchromane oder -chromene ausgewählt sind aus 3,4-Dihydro-7-methoxy-2,2-dimethyl-2H-1 -benzopyran-6-ol.

18. Verwendung gemäß Anspruch 1, **dadurch gekennzeichnet, dass** die Steroidsulfatase-inhibierende Substanz ausgewählt ist aus Aluminiumchlorohydrat.

19. Verwendung gemäß Anspruch 1, **dadurch gekennzeichnet, dass** die Tropolonderivate ausgewählt sind aus Hinokitiol.

20. Verwendung gemäß Anspruch 1, **dadurch gekennzeichnet, dass** die Ester der Schwefelsäure mit alkoxylierten C₈-C₁₈-Fettalkoholen und deren physiologisch verträgliche Metallsalze ausgewählt sind aus Zinkcocoylethersulfat mit einem Ethoxylierungsgrad n von 1 - 20.

21. Kosmetische oder pharmazeutische Zusammensetzung zur Verminderung von Haarausfall, **dadurch gekennzeichnet, dass** mindestens ein Ester der Schwefelsäure mit alkoxylierten C₈-C₁₈-Fettalkoholen und deren physiologisch verträgliche Metallsalze mit einem Alkoxylierungsgrad n von 1 - 30 enthalten ist.

22. Kosmetische oder pharmazeutische Zusammensetzung gemäß Anspruch 21, **dadurch gekennzeichnet, dass** Zinkcocoylethersulfat mit einem Ethoxylierungsgrad n von 1 bis 20 enthalten ist.

23. Verwendung gemäß Anspruch 1, **dadurch gekennzeichnet, dass** die Ester der Phosphorsäure und der Triphosphorsäure von ein- bis sechswertigen Hydroxyverbindungen ausgewählt sind aus den Phosphaten und Triphosphaten von alkoxylierten C₈-C₂₀-Alkanolen, Inositolen und N-Glykosiden.

24. Verwendung gemäß Anspruch 23, **dadurch gekennzeichnet, dass** die Phosphate von alkoxylierten C₈-C₂₀-Alkanolen ausgewählt sind aus den Phosphaten von linearen C₈-C₂₀-Alkanolen mit n Ethylenoxid- und m Propylenoxid-Einheiten mit n = 2 - 20 und m = 2 - 10.

25. Verwendung gemäß Anspruch 23, **dadurch gekennzeichnet, dass** die Phosphate und Triphosphate von Inositolen und N-Glykosiden ausgewählt sind aus Phytinsäure und Adenosintriphosphat.

26. Verwendung gemäß Anspruch 1, **dadurch gekennzeichnet, dass** die Kieselsäureester ausgewählt sind aus Verbindungen der Formeln IV, V, VI, VII und VIII. 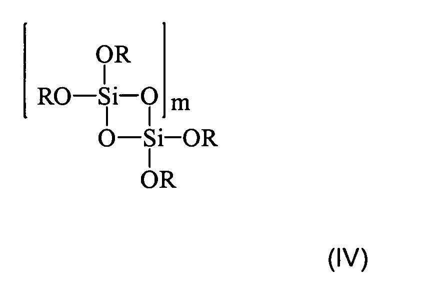 und 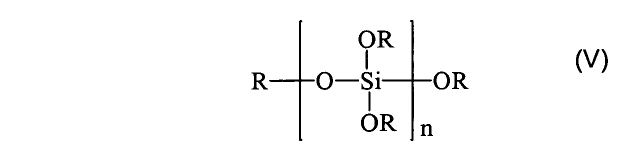 und 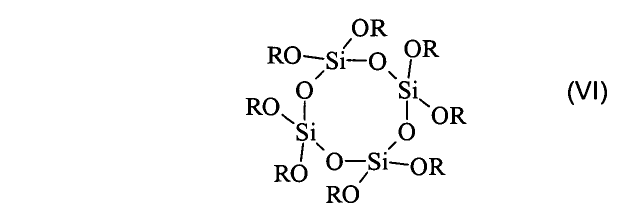 und 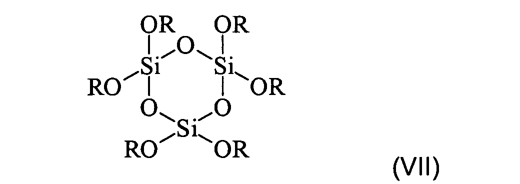 und 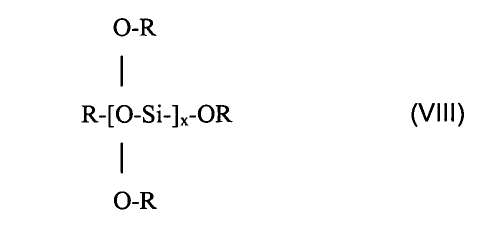 wobei alle R unabhängig voneinander ausgewählt sind aus der Gruppe, die ein Wasserstoffatom, die geradkettigen oder verzweigten, gesättigten oder ungesättigten, substituierten oder unsubstituierten C₁₋₆-Kohlenwasserstoffreste und den 2-Phenoxyethyl-Rest enthält, wobei mindestens ein R verschieden von einem Wasserstoffatom sein muss, und m Werte aus dem Bereich 1 bis 20 und n Werte aus dem Bereich 2 bis 100 und x Werte aus dem Bereich 2 bis 20 annimmt, sowie Mischungen hiervon.

27. Verwendung gemäß Anspruch 1, **dadurch gekennzeichnet, dass** die quaternären Siliconverbindungen ausgewählt sind aus Amodimethiconen, Trimethylsilylamodimethiconen und Quaternium-80.

28. Pharmazeutische Zubereitung zur Behandlung und/oder Prophylaxe von androgen-abhängigen Störungen des Haarfollikels, insbesondere von androgenetisch bedingtem Haarausfall, enthaltend Steroidsulfatase-Inhibitoren gemäß Anspruch 1.

29. Verfahren zur Verminderung von Haarausfall mittels Inhibierung von Steroidsulfatase, **dadurch gekennzeichnet, dass** eine kosmetische oder pharmazeutische Zusammensetzung, enthaltend mindestens eine Steroidsulfatase-inhibierende Substanz, ausgewählt aus funktionellen C₂-C₁₂-Carbonsäuren und deren physiologisch verträglichen Metallsalzen, Derivaten von C₃-C₉-Polyolen, Phenolderivaten, Pflanzenextrakten, Riechstoffen, 6,7-disubstituierten 2,2-Dialkylchromanen oder -chromenen, Flavonoiden, Isoflavonoiden, Aluminiumchlorohydrat, 2-Phenylethanol, Etidronsäure, 7-Acetyl-1,1,3,4,4,6-hexamethyltetralin, Tropolonderivaten, Estern der Schwefelsäure mit alkoxylierten C₈-C₁₈-Fettalkoholen und deren physiologisch verträglichen Metallsalzen, Estern der Phosphorsäure und der Triphosphorsäure mit ein- bis sechswertigen Hydroxyverbindungen, Kieselsäureestern, aus marinen Organismen isolierbaren mycosporin-ähnlichen Aminosäuren (MAA) sowie quaternären Siliconverbindungen, auf die Haut, insbesondere auf die Kopfhaut, aufgetragen wird.
